# FASCICULE DE BREVET EUROPEEN

(11) **EP 0 454 826 B1**
(45) Date de publication et mention de la délivrance du brevet: **31.01.1996**
(21) Numéro de dépôt: 90917219.9
(22) Date de dépôt: 16.11.1990
(51) Int. Cl.: G01N 33/543, G01N 35/02

(54) **APPAREIL D'EXECUTION AUTOMATIQUE D'UN IMMUNODOSAGE EN PLUSIEURS ETAPES SUCCESSIVES D'AU MOINS UNE SUBSTANCE BIOLOGIQUE DANS UNE PLURALITE D'ECHANTILLONS BIOLOGIQUES, PROCEDE ET REACTIF UTILISANT LEDIT APPAREIL**
AUTOMATISCHES GERÄT FÜR EINEN STUFENWEISEN IMMUNTEST MINDESTENS EINER BIOLOGISCHEN SUBSTANZ IN EINER MEHRZAHL VON PROBEN, VERFAHREN UND REAGENS DIE BESAGTES GERÄT GEBRAUCHEN
APPARATUS FOR AUTOMATICALLY CARRYING OUT A MULTI-STAGE IMMUNOASSAY OF AT LEAST ONE BIOLOGICAL SUBSTANCE IN A PLURALITY OF BIOLOGICAL SAMPLES; METHOD AND REAGENT USING SAID APPARATUS

(30) Priorité: 17.11.1989 FR 8915095
(43) Date de publication de la demande: 06.11.1991
(60) Demande divisionnaire: 95110811.7
(73) Titulaire: Laboratoires Merck-Clévenot, F-92736 Nogent-sur-Marne (FR)
(72) Inventeur: UZAN, Michel, F-93320 Les Pavillons-sous-Bois (FR); GICQUEL, Thierry, F-95000 Jouy-le-Moutier (FR); LENTWOJT, Edouard, F-60340 Saint-Leu-d'Esserent (FR); NARMINIO, Dario, F-95380 Louvres (FR)
(74) Mandataire: Ores, Irène
(86) Numéro de dépôt international: FR9000823
(87) Numéro de publication internationale: WO9107662

(56) Documents cités:
- EP-A- 0 038 730
- EP-A- 0 216 026
- EP-A- 0 249 357
- EP-A- 0 355 823
- WO-A-88/02866
- FR-A- 2 454 098
- CLINICA CHIMICA ACTA, vol. 84, 1978; D.S. ITHAKISIOS, pp. 69-84/

## Description

La présente invention est relative à un appareil d'exécution automatique d'un immunodosage en plusieurs étapes successives, d'au moins une substance biologique dans une pluralité d'échantillons biologiques ainsi qu'au procédé et aux réactifs pour l'utilisation dudit appareil.

L'invention s'applique plus particulièrement, mais non limitativement, à la détection simultanée sur un même échantillon de ligands, d'antiligands, d'haptènes ou de toute autre substance biologique éventuellement présente dans le fluide biologique à analyser.

Les méthodes de détermination de la présence ou de la concentration de ligands ou de substances biologiques dans des fluides biologiques, par voie immunologique, sont à présent bien connues ; elles sont basées sur la formation d'un complexe entre la substance à déterminer et un ou des anticorps, l'un des composants du complexe pouvant être marqué notamment par une enzyme, pour permettre sa détection et/ou son analyse quantitative après séparation de l'antigène ou de l'anticorps marqué complexé, de l'antigène ou de l'anticorps marqué non complexé.

Le principe des méthodes RIA et ELISA est également bien connu.

La technique ELISA (Enzyme linked immunosorbent assay), notamment, est une méthode immuno-enzymatique, d'une très grande sensibilité, de titrage d'anticorps, par exemple, qui consiste à utiliser un complexe immunoadsorbant constitué notamment par un antigène fixé sur un support solide, pour capter les anticorps spécifiques contenus dans le milieu biologique, tel que sérum, à tester, l'immun-complexe ainsi obtenu étant détecté par un anticorps anti-espèce marqué par une enzyme, après quoi on ajoute un substrat spécifique de l'enzyme, dont la dégradation par l'enzyme fait apparaître un produit coloré ; l'intensité de la coloration est proportionnelle à la quantité d'enzyme qui réagit et donc à la quantité d'anticorps présente dans le milieu biologique testé ; l'intensité de la coloration peut être appréciée à l'oeil nu ou mesurée par tout moyen approprié, notamment par photométrie.

Dans les méthodes immunologiques ci-dessus, utilisant une phase hétérogène, la nature de cette phase solide qui sert de support à la réaction immunologique est très importante car elle détermine la sensibilité de la mesure.

Les supports qui ont été proposés pour le test ELISA sont, notamment, des grains de polyacrylamide activés par le glutaraldéhyde (AVRAMEAS et TERNYNCK, Immunochemistry, 1969, 6, pages 53-65) ; des particules de cellulose activée par le bromure de cyanogène, sur lesquelles des anticorps sont fixés par liaisons covalentes (ENGVALL et PERLMANN, Immunochemistry, 1971, 8, 871-874) ; des tubes de polystyrène sur la surface desquels des antigènes sont fixés par simple adsorption physique (ENGVALL et PERLMANN, J. Immun. 1972, 160, 129-136) ; des disques de papier activé par le bromure de cyanogène et sur lesquels est fixé un anticorps ou un antigène (BRIGHTON et Coll. Scand. 1974, 29, 166-174). Cependant, les supports les plus répandus actuellement sont les surfaces en matière plastique qui se présentent sous forme àe microplaques de 96 puits à fond plat, en U ou en V, en polystyrène ou en PVC, ou de barrettes de 8 puits.

Un autre support, formé de billes magnétiques a également été décrit et permet d'envisager l'automatisation des procédés mis en oeuvre.

On peut citer notamment les billes magnétiques décrites dans le Brevet européen 38 730 RHONE POULENC SPECIALITES CHIMIQUES qui décrit des latex de polymères magnétiques, les billes magnétiques décrites dans le Brevet européen 125 995 ADVANCED MAGNETICS INC., les billes magnétiques décrites dans les Brevets français 2 262 805 et 2 454 098 CORNING GLASS WORKS ou les billes décrites dans les Demandes de Brevets européens SERONO DIAGNOSTICS PARTNERS 105 714, 190 006, 238 353, 249 357.

L'article paru dans Clin. Chim. Acta, 1978, 84, 69-84, est relatif à des dosages de T₄ (immunologiques et non immunologiques) utilisant des microparticules magnétiques d'albumine pour la séparation de la T₄ libre, dans un échantillon complexe. La T₄ libre se lie à l'albumine par adsorption sur les microparticules magnétiques d'albumine.

Les différentes billes magnétiques décrites dans les documents précités sont également applicables pour la mise en oeuvre d'un dosage immunologique tel que l'ELISA ou le RIA.

Tant la méthode RIA que la méthode ELISA peuvent être mises en oeuvre de plusieurs manières :
- La méthode indirecte est la plus simple pour doser des anticorps. L'antigène est fixé sur la micro-plaque (en présence d'albumine pour bloquer les sites non occupés). Le sérum à doser est ajouté et la fixation des anticorps est détectée par l'addition d'anticorps anti-Ig marqués convenablement, notamment couplés à une enzyme et révélés quantitativement au spectrophotomètre après addition du substrat de l'enzyme.
- Une technique de compétition peut être utilisée pour doser les antigènes. Comme dans la technique précédente, c'est l'antigène qui est toujours fixé sur les plaques. Un mélange d'antigène et de faibles quantités d'anticorps est ajouté. La quantité d'anticorps fixée sur la plaque est d'autant plus faible que les molécules d'antigène ont neutralisé plus de molécules d'anticorps.
- Des méthodes en sandwich ou bi-site peuvent être utilisées pour doser des antigènes ou des anticorps. Pour doser des antigènes, les microplaques sont recouvertes de l'anticorps, l'antigène est ajouté puis l'anticorps spécifique, marqué de manière appropriée, notamment couplé à une enzyme, est appliqué et révélé par l'addition du substrat. La technique ne s'applique cependant qu'aux antigènes divalents ou possédant plusieurs déterminants. Pour doser des anticorps, les microplaques sont au contraire recouvertes de l'antigène et on ajoute successivement l'anticorps à doser et l'antigène marqué par une enzyme.

De tels procédés ne sont toutefois pas entièrement automatisables car ils ne permettent pas de mettre en oeuvre un dosage automatique d'au moins une substance dans une pluralité d'échantillons. En effet, la mise en oeuvre de ces différents procédés ne permet d'effectuer que des dosages en série de même type (compétition ou sandwich ou immunocapture) paramètre par paramètre, soit en flux continu, soit en flux discontinu, à cadences faibles.

La Demande WO 88/02866 décrit un appareil pour des dosages immunologiques en phase hétérogène, automatisés, mettant en oeuvre un disque de transfert contenant les tubes de réaction et permettant de réaliser un grand nombre de tests de différentes sortes en faisant varier les périodes d'incubation en fonction de l'analyte et du réactif (page 3, lignes 1-13).

La Demande EP 355 823 décrit également un appareil pour des dosages immunologiques en phase hétérogène, automatisés, ledit appareil étant propre à recevoir une pluralité de tubes de réactions et ayant une pluralité de positions de travail localisées séquentiellement, des moyens de positionnement séquentiel et pas-à-pas des tubes de réaction, lesdits moyens de positionnement effectuant au moins deux cycles complets.

Les différentes positions sont associées à des moyens convenables pour ajouter un échantillon et/ou des réactifs, pour incuber, pour laver ou pour mesurer le contenu des tubes, comprenant notamment des moyens de lavage sous la forme de deux blocs séparés, mobiles selon un axe horizontal et qui peuvent être désactivés selon le cas ; un tel dispositif permet en particulier de réaliser un grand nombre de tests différents avec différents protocoles.

Or, il est souhaitable, notamment dans les laboratoires d'analyses médicales de fournir des dispositifs automatiques de dosages multi-paramétriques à accès aléatoire, beaucoup plus rapides à réaliser et d'un moindre coût.

Un dosage multi-paramétrique à accès aléatoire permet, notamment sur un échantillon biologique, de réaliser le dosage de vingt paramètres différents et sur l'échantillon suivant de ne réaliser le dosage que de deux paramètres par exemple.

La Demanderesse s'est en conséquence donné pour but de pourvoir à un procédé et à un appareil de détermination et/ou de dosage automatique d'au moins une substance, notamment ligands, antiligands, haptènes ou autres molécules biologiques, qui répondent mieux aux nécessités de la pratique que les appareils et procédés de l'Art antérieur, notamment en ce qu'ils permettent un dosage multiparamétrique, à accès aléatoire, pour chaque échantillon, dans un temps relativement court (cadence élevée d'exécution des dosages) et à un moindre coût, en ce qu'ils permettent d'utiliser beaucoup moins de réactifs, et plus particulièrement des "monoréactifs", d'emploi plus simple, et en ce qu'ils permettent la réalisation de différents types de dosage, tels que compétition, bi-site, immunocapture.

La présente invention a pour objet un dispositif de lavage utilisable dans la mise en oeuvre d'un procédé de dosage de type immunologique en phase hétérogène, du type comprenant un support solide sous forme de billes magnétiques, caractérisé en ce qu'il comprend :
(1) quatre moyens d'application d'un champ magnétique à la partie inférieure des tubes (Tr) de réaction contenant l'échantillon à analyser et un ou plusieurs réactifs appropriés aux dosages à réaliser, l'un desdits réactifs consistant en billes magnétiques recouvertes d'une substance se liant spécifiquement à la substance à détecter, en suspension dans un liquide approprié et constituées d'une matrice organique renfermant une charge magnétique, lesdites billes ayant un rapport masse de matière magnétisable/masse de matière non magnétisable supérieur ou égal à 45 %, chaque moyen d'application d'un champ magnétique consistant de préférence en une paire d'aimants disposés face à face et de part et d'autre de deux tubes de réactions consécutifs, chaque paire d'aimants (50) étant associée à une armature métallique de canalisation des lignes de flux magnétique, pour que l'application du champ magnétique soit limitée uniquement à deux tubes consécutifs à la fois ; et
(2) une tête de lavage (L) comprenant sur un support :
   (i) quatre moyens d'aspiration du liquide contenu dans lesdits tubes,
   (ii) trois moyens de distribution d'un liquide de lavage auxdits tubes,
   (iii) un moyen de distribution de substrat approprié,
lesdits moyens étant répartis sur ledit support, de telle sorte que le premier moyen soit nécessairement un moyen d'aspiration et le dernier moyen, un moyen de distribution du substrat,
lequel dispositif de lavage étant mobile selon son axe vertical.

La présente invention a également pour objet un appareil de dosage automatique de type immunologique d'au moins une substance dans une pluralité d'échantillons à analyser, en plusieurs étapes successives constituant un cycle analytique, en phase hétérogène, du type comprenant un support solide sous forme de billes magnétiques, lequel appareil comprend :
(1) un module échantillons (A) constitué par une pluralité de supports de tubes (Te) contenant lesdits échantillons ;
(2) un module réactionnel (C) constitué par :
   (i) une pluralité de supports de tubes (Tr) destinés à recevoir successivement une quantité aliquote desdits échantillons et une quantité aliquote d'un réactif approprié, et
   (ii) une pluralité de positions de traitement séquentielles desdits tubes de réaction, constituant un cycle analytique ;
(3) un module réactifs (E) constitué par une pluralité de supports de tubes contenant le/les réactifs appropriés aux différents dosages à réaliser, au moins l'un desdits réactifs étant sous forme de billes magnétiques recouvertes avec une substance se liant spécifiquement à la substance à détecter, en suspension dans un liquide approprié ;
(4) un moyen de prélèvement et de distribution (B) des échantillons dans les tubes (Tr) dudit module réactionnel ;
(5) un moyen de prélèvement et de distribution (D) des réactifs dans les tubes dudit module réactionnel ;
(6) un moyen approprié de lecture de la réaction effectuée dans le module réactionnel ; lequel appareil est caractérisé en ce que :
   (a) ledit module de réaction (C) connecté à un microprocesseur de contrôle, inclut un dispositif, selon la revendication 1, de lavage desdites billes magnétiques, constituées d'une matrice organique renfermant une charge magnétique, lesdites billes ayant un rapport masse de matière magnétisable/masse de matière non magnétisable supérieur ou égal à 45 %, et
   (b) en ce que ledit appareil comprend un système informatique constitué par un ordinateur de commande des différents modules et moyens (1) à (6), et permettant l'exécution d'une succession de cycles analytiques.

Un cycle analytique selon l'invention comprend une série d'étapes constituant au moins une incubation immunologique, et une série d'étapes constituant une incubation de révélation, notamment une incubation enzymatique ; chaque étape correspond à un nombre de positions séquentielles d'un tube dans le module réactionnel, le temps d'arrêt d'un tube dans une position étant constant.

Selon une caractéristique de l'invention, la tête de lavage du dispositif fixe de lavage du module réactionnel comprend comme moyens d'aspiration et de distribution, un support muni d'aiguilles d'aspiration et de distribution d'une solution appropriée, lequel support est mobile selon un axe vertical, son déplacement étant synchronisé avec le déplacement des tubes de réaction.

Selon une autre caractéristique de l'appareil conforme à l'invention, les modules réactionnel, échantillons et réactifs comprennent chacun une couronne entraînée en rotation, lesdites rotations des différentes couronnes étant synchronisées par le système informatique.

Selon encore une autre caractéristique de l'appareil conforme à l'invention, ledit module réactionnel comprend un nombre de positions de tubes correspondant à la réalisation d'au moins un cycle analytique comportant la série d'étapes successives suivantes :
- dépôt de l'échantillon à analyser en posi tion 1 ;
- dépôt du réactif approprié en position 4 ;
- préaimantation en position 86 ;
- aimantation et aspiration en position 87 ;
- lavage en position 88 ;
- préaimantation en position position 89 ;
- aimantation et aspiration en position 90 ;
- lavage en position 91 ;
- préaimantation en position 92 ;
- aimantation et aspiration en position 93 ;
- lavage en position 94 ;
- préaimantation en position 95 ;
- aimantation et aspiration en position 96 ;
- distribution éventuelle du substrat en position 98 ;
- préaimantation en position 82 ;
- lecture du résultat en position 83.

On définit, au sens de la présente invention, l'opération de lavage comme étant un ensemble d'étapes obligatoires de l'incubation immunologique, à savoir les étapes successives de préaimantation des billes magnétiques, d'aimantation desdites billes, réalisée simultanément à l'aspiration du surnageant et de distribution de solution de lavage.

Le dispositif de lavage conforme à l'invention permet de réaliser plusieurs opérations de lavage simultanément sur différents tubes de réaction.

Certaines des étapes précisées ci-dessus sont donc obligatoires, tel l'ensemble des étapes formant l'opération de lavage ; d'autres étapes, telle que la distribution d'un substrat, peuvent s'avérer facultatives, selon le marqueur choisi.

On définit, au sens de la présente invention, une rotation complète du module réactionnel, comme étant la succession d'un nombre donné de positions prises par un tube.

Si l'on considère la position 1 comme étant la position de départ de chaque tube et le nombre de positions comme étant par exemple de 100, une rotation complète comprend 100 positions successives pour un tube donné.

L'ensemble de ces étapes successives constitue comme défini ci-dessus, un cycle analytique, qui comprend, dans le cas de la distribution d'un seul réactif (dosage "monoréactif"), deux rotations complètes du module réactionnel, par tout moyen approprié, notamment un moteur électrique et dans le cas de la distribution de deux réactifs (dosage "bi-réactif"), trois rotations complètes du module réactionnel, la lecture du résultat étant toujours réalisée à la fin de la dernière rotation.

Selon encore une autre caractéristique de l'invention, le temps d'arrêt d'un tube de réaction dans une position est compris entre 5 et 20 secondes environ.

On peut ainsi réaliser une centaine de séquences analytiques à l'heure.

Selon une autre caractéristique de l'appareil conforme à l'invention, le module réactionnel est, en outre, associé à un dispositif de régulation de la température.

Selon encore une autre caractéristique de l'invention, l'appareil comprend en outre au moins un moyen de décontamination par rinçage des moyens de prélèvement et de distribution des échantillons et des réactifs.

Ce moyen de décontamination est avantageusement constitué par un récipient associé, par une pompe et une électrovanne trois voies, par exemple, à un réservoir de détergent approprié.

L'appareil conforme à l'invention a l'avantage de permettre l'automatisation intégrale des techniques ELISA, RIA, immunofluorescence, etc..., et de réaliser simultanément sur un même échantillon, par exemple des dosages de protéines, d'haptènes et d'anticorps, en un temps très court inférieur à 1 heure.

La présente invention a également pour objet un procédé de dosage immunologique en phase hétérogène, notamment par compétition ou par une méthode bi-site, en plusieurs étapes successives, d'au moins une substance dans une pluralité d'échantillons à analyser, utilisant comme support solide des billes magnétiques appropriées, du type comprenant des étapes :
- de distribution successive d'échantillons et de réactifs,
- d'incubation immunologique,
- de lavage et
- de mesure de ladite substance,
lesquelles étapes étant automatisées, constituant un cycle analytique incluant une pluralité de positions de traitement séquentielles desdits tubes réactifs et étant déterminées par les rotations synchrones de modules échantillons, réactifs et réactionnel, portant respectivement les tubes contenant les échantillons à analyser, les tubes réactifs et les tubes de réaction,
lequel procédé est caractérisé en ce que :
(a) lesdites billes magnétiques, recouvertes d'une substance se liant spécifiquement à la substance à détecter, en suspension dans un liquide approprié, sont constituées d'une matrice organique renfermant une charge magnétique, et ayant un rapport masse de matière magnétisable/masse de matière non magnétisable supérieur ou égal à 45 %,
(b) ladite incubation immunologique est de durée identique quelle que soit la substance à déterminer et est égale à une rotation du module de réaction entre les positions de traitement 5 et 85,
(c) le lavage desdites billes magnétiques est mis en oeuvre à l'aide d'un dispositif de lavage selon la revendication 1, et
(d) l'étape de mesure comprend la séquence suivante de positions de traitement :
   position 95 : pré-aimantation
   position 96 : aimantation et aspiration
   position 98 : distribution du substrat
   position 82 : pré-aimantation
   position 83 : lecture du résultat.
   position 82 : pré-aimantation
   position 83 : lecture du résultat.

La durée d'une incubation immunologique, dans le procédé conforme à l'invention est égale à une rotation complète du module réactionnel ; la durée d'une incubation de révélation est également de l'ordre d'une rotation complète du module réactionnel et dépend de la durée de rotation entre la distribution du substrat et l'étape de lecture du résultat ; lorsque le procédé comprend, par exemple, deux incubations immunologiques, le module réactionnel est soumis à trois rotations avant que la lecture du résultat puisse être réalisée.

Selon un mode de mise en oeuvre avantageux du procédé conforme à l'invention, le cycle analytique comprend une seule incubation immunologique et une incubation de révélation constituée par une incubation enzymatique, lesquelles incubations comprennent les étapes suivantes :
I. incubation immunologique :
   a - la distribution d'une quantité aliquote d'échantillon à analyser dans un tube de réaction ;
   b - la distribution d'une quantité aliquote de réactif approprié dans ledit tube de réaction ;
   c - la mise en contact de l'échantillon et du réactif pendant un temps approprié, correspondant à la durée d'une rotation complète d'un tube de réaction ;
   d - une première sédimentation des billes magnétiques suivie d'une aspiration du surnageant puis d'un premier lavage ;
   e - une deuxième sédimentation des billes magnétiques suivie d'une aspiration du surnageant puis d'un deuxième lavage ;
   f - une troisième sédimentation des billes magnétiques suivie d'une aspiration du surnageant puis d'un troisième lavage ;
   g - une quatrième sédimentation des billes magnétiques suivie d'une aspiration du surnageant puis
II. incubation enzymatique
   h - une distribution du substrat et la mise en contact des billes magnétiques avec ledit substrat, pendant un temps approprié, dépendant de la durée de rotation dudit tube de réaction entre la distribution du substrat et l'étape de lecture du résultat, suivie d'une sédimentation desdites billes magnétiques ;
   i - puis la lecture du résultat ;
   chacune desdites étapes correspondant à un nombre de positions séquentielles d'un tube de réaction.

Un tel procédé est dénommé ci-après "procédé monoréactif".

Selon une disposition avantageuse de ce mode de mise en oeuvre, l'incubation est réalisée en présence d'un monoréactif constitué d'un prémélange d'un premier réactif R₁ comprenant des billes magnétiques recouvertes d'une substance S₁ se liant spécifiquement à la substance à détecter et d'un second réactif R₂ appelé "conjugué" constitué par un marqueur approprié couplé à une substance S₂, identique ou différente de S₁ et qui ne réagit pas avec S₁.

Selon une modalité avantageuse de cette disposition, ledit monoréactif est constitué d'un prémélange d'un premier réactif R₁ comprenant des billes magnétiques recouvertes d'une substance S₁ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser, et les antigènes appropriés notamment la substance à doser ou l'un de ses fragments et d'un second réactif R₂ ou "conjugué" constitué par de la phosphatase alcaline associée à une substance S₂ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser et les antigènes appropriés, notamment la substance à doser ou l'un de ses fragments et les complexes antigène-immunoglobuline.

Un tel réactif a l'avantage d'être stable pendant 30 jours ou plus et d'être particulièrement bien adapté au procédé de dosage automatique conforme à l'invention.

Selon un autre mode de mise en oeuvre du procédé conforme à l'invention, le cycle analytique comprend deux incubations immunologiques et une incubation de révélation constituée par une incubation enzymatique, lequel cycle comprend les étapes suivantes :
I. première incubation immunologique :
   a - le dépôt d'une quantité aliquote d'échantillon à analyser, dans un tube de réaction ;
   b - le dépôt d'une quantité aliquote de réactif approprié dans ledit tube de réaction ;
   c - la mise en contact de l'échantillon et du réactif pendant un temps approprié, correspondant à la durée d'une rotation complète d'un tube de réaction ;
   d - une première sédimentation des billes magnétiques suivie d'une aspiration du surnageant puis d'un premier lavage ;
   e - une deuxième sédimentation des billes magnétiques suivie d'une aspiration du surnageant puis d'un deuxième lavage ;
   f - une troisième sédimentation des billes magnétiques suivie d'une aspiration du surnageant puis d'un troisième lavage ;
   g - une quatrième sédimentation des billes magnétiques suivie d'une aspiration du surnageant puis
II. deuxième incubation immunologique
   - les étapes b- à g- sont réalisées une seconde fois ; puis
III. incubation enzymatique
   h - une distribution du substrat et la mise en contact des billes magnétiques avec ledit substrat, pendant un temps approprié, dépendant de la durée de rotation dudit tube de réaction entre la distribution du substrat et l'étape de lecture du résultat, suivie d'une sédimentation des billes magnétiques ;
   i - puis la lecture du résultat ; chacune desdites étapes correspondant à un nombre de positions séquentielles d'un tube de réaction.

Un tel procédé est dénommé ci-après "procédé bi-réactif".

Selon une disposition avantageuse de ce mode de mise en oeuvre, la première incubation immunologique est effectuée en présence d'un premier réactif R₁ constitué de billes magnétiques recouvertes d'une substance S₁ se liant spécifiquement à la substance à détecter et la seconde incubation immunologique est effectuée en présence d'un second réactif R₂ appelé "conjugué", constitué par un marqueur approprié couplé à une substance S₂, identique ou différente de S₁ et qui ne réagit pas avec S₁.

Selon une modalité avantageuse de cette disposition, la première incubation immunologique est effectuée en présence d'un premier réactif R₁, constitué de billes magnétiques recouvertes d'une substance S₁ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser, et les antigènes appropriés, notamment la substance à doser ou l'un de ses fragments et la seconde incubation immunologique est effectuée en présence d'un second réactif R₂ ou conjugué constitué par de la phosphatase alcaline associée à une substance S₂ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser et les antigènes appropriés, notamment la substance à doser ou l'un de ses fragments et les complexes antigène-immunoglobuline.

Pour certaines des substances à doser, un cycle analytique comprenant une seule incubation immunologique et une incubation de révélation est nécessaire ; il s'agit en particulier de l'α-féto-protéine (AFP), de la LH, de la FSH, de l'hCG, des IgE et de la prolactine et ce de manière non limitative.

Pour d'autres substances, le cycle analytique comprend deux incubations immunologiques et une incubation de révélation ; il s'agit notamment, et ce de manière non limitative, de la TSH, de la T3, de la T4 totale et de la T4 libre et du cortisol ou bien des anticorps qui apparaissent lors des infections, notamment dues à un parasite (toxoplasmose par exemple), un virus (rubéole, VIH, par exemple) ou à un microorganisme *(Chlamydia* par exemple).

Pour ces dernières substances (anticorps), le procédé conforme à l'invention permet de réaliser les dosages en évitant l'étape de pré-dilution habituellement nécessaire.

Selon encore un autre mode de mise en oeuvre du procédé conforme à l'invention, il comprend en outre une étape de calibration multiparamétrique, notamment réalisée préalablement au dosage des échantillons.

Le procédé conforme à l'invention présente un certain nombre d'avantages :
- il ne nécessite pas une calibration pour chaque série de dosages, une calibration des différents lots de réactifs étant suffisante ;
- il permet également de calibrer, lors d'une étape unique, l'ensemble des substances pouvant éventuellement être dosées ;
- il permet, de plus, un dosage multiparamétrique pour un échantillon,
- il permet également d'obtenir une cadence de dosage particulièrement élevée (par exemple 100 dosages en 50 minutes).

La présente invention a, de plus, pour objet un réactif de diagnostic immunologique constitué de billes magnétiques recouvertes d'une substance se liant spécifiquement à la substance à détecter, en suspension dans un liquide approprié, caractérisé en ce que lesdites billes sont constituées d'une matrice organique renfermant une charge magnétique, lesdites billes ayant un rapport masse de matière magnétisable/masse de matière non magnétisable supérieur ou égal à 45 %.

Selon un mode de réalisation particulièrement avantageux dudit réactif, il est constitué de billes magnétiques dont le rapport masse de matière magnétisable/masse de matière non magnétisable est compris entre 60 et 70 %.

De manière particulièrement avantageuse, la matrice organique est un latex de polymères tel que de polystyrène ou de divinylbenzène.

De préférence, ledit réactif est constitué de billes magnétiques ayant un diamètre inférieur à 1,5 µm et plus particulièrement compris entre 0,7 et 1,5 µm.

On peut citer notamment comme billes magnétiques présentant de telles propriétés, les particules décrites dans le Brevet européen 38730, au nom de RHONE-POULENC SPECIALITES CHIMIQUES et dénommées "billes ESTAPOR".

De manière particulièrement avantageuse, ledit réactif est constitué de billes magnétiques recouvertes d'une substance S₁ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à détecter, et les antigènes appropriés, notamment la substance à détecter ou l'un de ses fragments.

Le couplage de ladite substance sur de telles particules magnétiques peut être réalisé selon les techniques de couplage par liaisons covalentes décrites dans l'Art antérieur et notamment selon le mode opératoire décrit par WOOD et coll. dans le Journal of Clinical Chemistry and Clinical Biochemistry, vol. 21, 1983, pp. 789-797.

Selon un autre mode de réalisation dudit réactif, il est constitué d'un prémélange comprenant lesdites billes magnétiques recouvertes d'une substance S₁ et un conjugué constitué par un marqueur approprié couplé à une substance S₂, identique ou différente de S₁ et qui ne réagit pas avec S₁, lequel prémélange est réalisé pour la mise en oeuvre du procédé conforme à l'invention.

Selon une disposition avantageuse de ce mode de réalisation, le réactif comprend un conjugué constitué par de la phosphatase alcaline associée à une substance S₂ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser et les antigènes appropriés, notamment la substance à doser ou l'un de ses fragments et les complexes antigène-immunoglobuline.

Ledit réactif est notamment constitué à'une solution contenant 0,1 % en rapport P/V de billes magnétiques (soit 1 g en poids sec de billes magnétiques dans 1 litre d'un tampon approprié).

Un tel réactif est particulièrement bien adapté à la mise en oeuvre automatique sur un appareil tel que décrit ci-dessus, du procédé conforme à l'invention, car tout en étant constitué d'une phase solide, il est manipulable comme un liquide, permettant ainsi un gain de temps et une simplicité, dans l'exécution des différentes opérations nécessaires aux techniques de dosage immunologique et notamment à la méthode ELISA.

Par ailleurs, la charge magnétique élevée des billes qui le constituent autorise, sous l'action d'un champ magnétique approprié, une durée d'aimantation extrêmement courte, permettant la séparation totale desdites billes du surnageant. A titre d'exemple, cette durée d'aimantation est de 15 secondes sous l'action d'un champ magnétique constitué de deux aimants de 1023 tesla (ou Wb.m⁻) environ, autorisant une cadence élevée d'exécution des dosages sur l'analyseur automatique.

De plus, la surface réactionnelle importante des billes magnétiques permet, quelle que soit la substance à doser, que la durée d'une incubation immunologique soit toujours égale à une rotation complète du module réactionnel.

La présente invention a en outre pour objet un kit ou trousse de diagnostic, prêt à l'emploi, caractérisé en ce qu'il est constitué par au moins un réactif de diagnostic immunologique conforme à l'invention.

Outre les dispositions qui précèdent, l'invention comprend encore d'autres dispositions qui ressortiront de la description qui va suivre, faite en référence aux dessins annexés dans lesquels la figure 1 illustre schématiquement l'appareil conforme à l'invention ; la figure 2 est une vue schématique en perspective de la tête de lavage ; la figure 3 illustre schématiquement le fonctionnement du dispositif de lavage ; la figure 4 représente schématiquement la succession des étapes réalisées au niveau du module réactionnel ; la figure 5 est une représentation schématique du dispositif de décontamination.

Il doit être bien entendu, toutefois, que ces dessins et les parties descriptives correspondantes sont donnés uniquement à titre d'illustration de l'objet de l'invention, dont ils ne constituent en aucune manière une limitation.

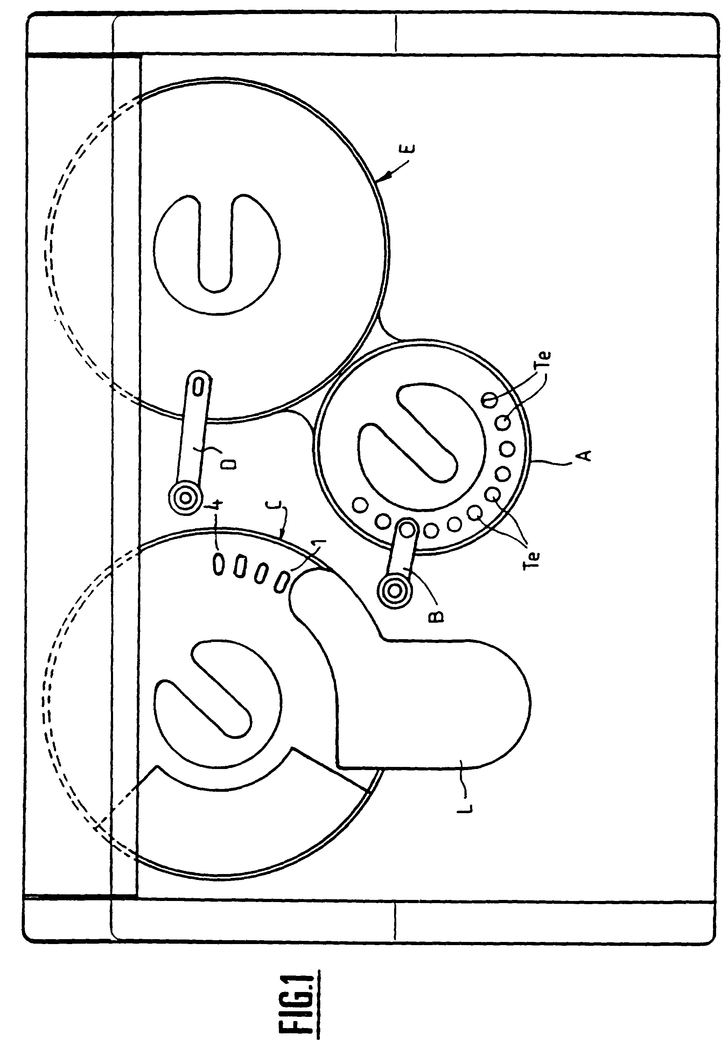

L'appareil conforme à l'invention est illustré schématiquement sur la figure 1.

Il comprend un module échantillons A, un module réactionnel C et un module réactifs E, chaque module étant constitué essentiellement d'une couronne équipée de supports de tubes, amovibles et entraînée en rotation par un moteur électrique sous commande d'un microprocesseur. Une unité de commande constituée d'un ordinateur assure la coordination du fonctionnement, et notamment des rotations des trois modules A, C et E de telle sorte qu'elles soient synchronisées.

Le module échantillons A comprend un support destiné à recevoir une pluralité de tubes Te disposés en couronne et contenant chacun un échantillon de fluide biologique dans lequel au moins une substance est à doser. Avantageusement, le support peut comporter une deuxième série d'emplacements destinés à recevoir des godets contenant des solutions de calibration.

Le module échantillons A est associé à un bras B de prélèvement et de distribution des échantillons.

Le module réactifs E supporte une pluralité de tubes ou godets contenant les réactifs appropriés aux différents dosages à réaliser, et est associé à un bras D de prélèvement et de distribution desdits réactifs.

Le module réactionnel C supporte une pluralité de tubes de réaction et comprend un dispositif de lavage fixe constitué d'une tête de lavage L et d'au moins un moyen d'application d'un champ magnétique à la partie inférieure des tubes de réaction.

Dans un mode de réalisation préféré de l'appareil selon l'invention, la tête de lavage L est constituée d'un support d'aiguilles d'aspiration et de distribution, ledit support reposant sur une potence et étant mobile selon un axe vertical.

De préférence, et ce de manière non limitative, le support d'aiguilles est muni de quatre aiguilles d'aspiration, de trois aiguilles de distribution d'une solution de lavage et d'une aiguille de distribution d'un substrat.

Le module réactionnel C peut avantageusement être thermostaté à 37°C.

Dans un mode de réalisation préféré de cet appareil, le module échantillons A peut comprendre jusqu'à 36 tubes Te, notamment des tubes à prélèvement tels qu'habituellement utilisés dans les laboratoires d'analyses médicales et jusqu'à 18 godets de calibration. Le module réactifs E peut comprendre jusqu'à 20 godets pouvant contenir jusqu'à 100 ml de réactif et le module réactionnel C supporte 100 tubes réactionnels (4 plateaux de 25 cuvettes) pouvant donc occuper 100 positions angulaires différentes sur lesquelles ils marquent un arrêt de durée constante qui est avantageusement de 10 secondes.

Le bras B de prélèvement et de distribution des échantillons est constitué d'un support d'aiguille reposant sur une potence, d'une aiguille de diamètre approprié et d'un détecteur capacitif de niveau, qui permet, quel que soit le volume d'échantillon contenu dans un tube Te, de faire plonger l'extrémité de l'aiguille dans l'échantillon d'une hauteur prédéterminée et constante. Dans un mode de réalisation préféré de l'appareil, le volume d'échantillon pouvant être prélevé par le bras B de prélèvement et de distribution des échantillons est compris entre 10 et 150 µl.

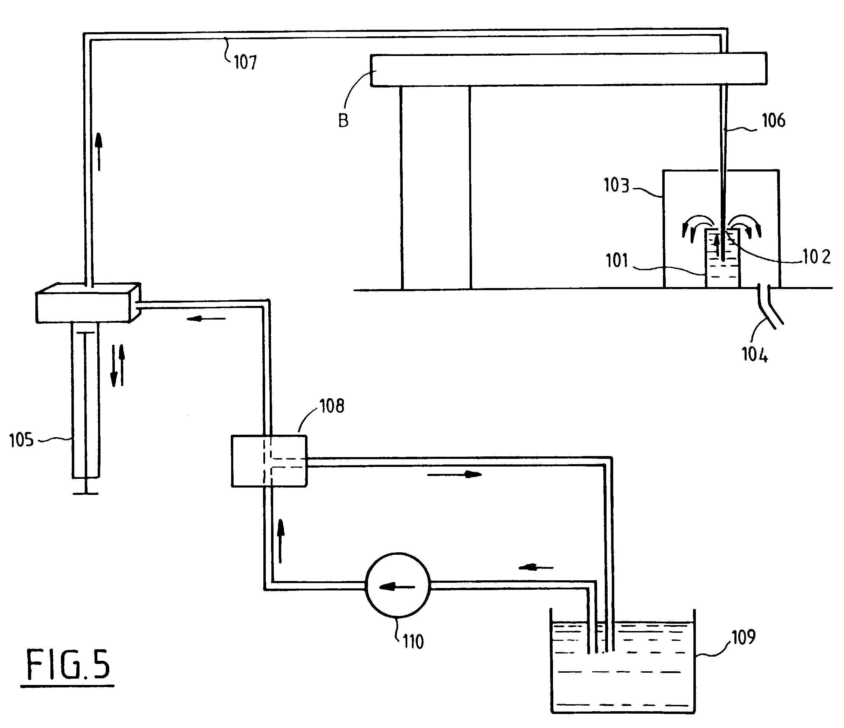

Avantageusement, ledit bras B est muni d'un dispositif de décontamination interne et externe de l'aiguille entre deux prélèvements, comprenant (cf. figure 5) :
- un plot de décontamination 101 comportant une ouverture 102 à sa partie supérieure, contenant le liquide de décontamination, et inclus dans une enceinte 103 munie d'un moyen de vidange 104,
- une seringue 105, reliée d'une part à la partie supérieure de l'aiguille 106 à décontaminer, par un tuyau souple 107 et d'autre part, à une électrovanne 3 voies 108, elle-même reliée en boucle à un bidon de solution de décontamination 109 et à une pompe péristaltique 110.

Après avoir effectué le dépôt d'une quantité aliquote d'échantillon dans un tube réactionnel Tr, le bras B pivote pour amener l'aiguille à décontaminer dans le plot de décontamination ; lorsque l'électrovanne s'ouvre, la solution de décontamination contenue dans le plot de décontamination passe, pendant une seconde, dans l'aiguille à décontaminer ; lorsque l'électrovanne se referme, la seringue aspire un volume, par exemple de 200 µl de solution de décontamination, puis l'électrovanne s'ouvre et simultanément la seringue éjecte sous pression 150 µl et garde 50 µl de solution de décontamination, lesquels serviront de complément volumique au prochain aliquote d'échantillon devant être prélevé, puis déposé dans un tube réactionnel ; l'aiguille éjecte le liquide de décontamination avec une pression suffisante pour permettre audit liquide de remonter pour laver l'aiguille extérieurement. Le liquide se vide dans l'ouverture du plot et le surplus s'évacue par le moyen de vidange de l'enceinte ; l'électrovanne se referme, le bras B se déplace pour sortir l'aiguille du plot de décontamination et l'amener au dessus de l'échantillon à prélever sur le module échantillons A.

Pendant le lavage, la seringue agit sur le débit de solution de décontamination et favorise ainsi le lavage.

Le bras B prélève un aliquote dudit échantillon et le dépose, avec le complément volumique retenu dans la seringue, dans le tube de réaction situé en position 1 du module réactionnel C.

Avantageusement, la solution de décontamination est un détergent connu par exemple du "Tween".

Les trois opérations prélèvement de l'échantillon, distribution de l'échantillon et décontamination de l'aiguille, commandées par le microprocesseur du module échantillons s'effectuent pendant le temps d'arrêt d'un tube réactionnel en position 1 du module réactionnel, soit avantageusement pendant 10 secondes.

Si le dosage suivant porte sur le même échantillon, seul le module réactionnel C avance d'un tube, ledit module échantillons A restant immobile. Dans le cas contraire, les deux modules réactionnel C et échantillons A avancent conjointement d'un tube. La distribution d'échantillon en position 1 du module réactionnel est répétée pour tous les échantillons présents sur le module échantillons, et pour un échantillon donné, pour toutes les substances à doser dans ledit échantillon.

Le module réactifs E est muni d'un bras D de prélèvement et de distribution des réactifs, sensiblement analogue au bras B de prélèvement et de distribution des échantillons et pouvant être avantageusement muni d'un dispositif de décontamination comparable. Dans un mode de réalisation préféré de l'appareil selon l'invention, le volume de réactif pouvant être prélevé et distribué est compris entre 50 et 400 µl.

Lorsque le premier tube réactionnel dans lequel a été réalisé le dépôt d'un aliquote d'échantillon arrive en position 4 du module réactionnel, le bras D de prélèvement des réactifs prélève au niveau du module réactifs E un aliquote du réactif approprié et le dépose dans ledit tube réactionnel en position 4. Le module réactionnel avance alors d'une position pour amener le tube réactionnel suivant en position 4. La rotation du module réactifs E, indépendante de celle des deux autres modules s'effectue en fonction du réactif devant être déposé dans chacun des tubes arrivant successivement en position 4 du module réactionnel C et contenant un aliquote d'échantillon à doser.

Le module réactifs E peut avantageusement comporter des moyens d'agitation des réactifs, permettant le maintien en suspension des billes magnétiques les constituant et garantissant ainsi leur homogénéité. L'agitation des réactifs est notamment réalisée par une rotation alternative de la couronne constituant le module réactifs, commandée par le microprocesseur dudit module et se produisant pendant 20 secondes toutes les 10 minutes lorsque l'appareil est en veille, et de manière continue pendant que s'effectuent les prélèvements et distributions des réactifs.

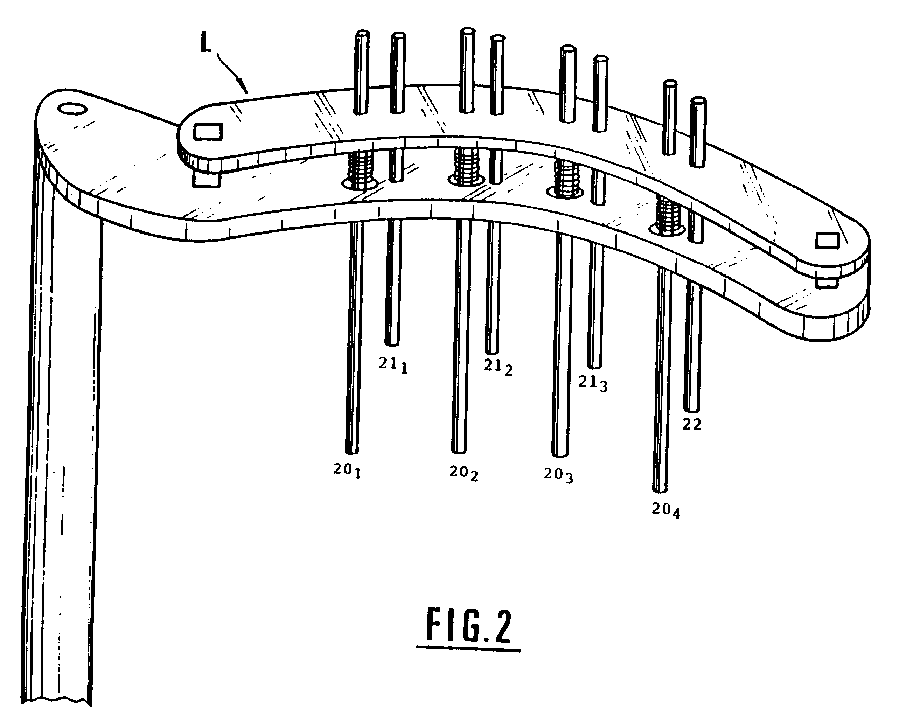

La figure 2 représente une tête de lavage L conforme à l'invention, associée au module réactionnel C et qui comprend un support d'aiguilles qui porte avantageusement 4 aiguilles d'aspiration 20₁-20₄, 3 aiguilles de distribution d'une solution de lavage 21₁-21₃ et 1 aiguille de distribution d'un substrat 22, lesdites aiguilles étant disposées dans un ordre déterminé.

Les quatres aiguilles d'aspiration 20₁-20₄ sont reliées à leur partie supérieure à deux pompes par un tuyau souple, lesquelles sont situées en amont d'un flacon de recueil.

Les trois aiguilles de distribution de la solution de lavage sont reliées à leur partie supérieure à 3 électrovannes 3 voies par un tuyau, lesquelles sont elles-mêmes reliées d'une part à un collecteur, d'autre part à une pompe péristaltique, eux-mêmes reliés à un réservoir contenant une solution de lavage.

L'aiguille de distribution de substrat est reliée à sa partie supérieure à une électrovanne, reliée à un flacon de substrat.

Le déplacement vertical au support d'aiguilles et la synchronisation du fonctionnement des différents moyens constituant la tête de lavage L sont commandés par le microprocesseur du module réactionnel C.

Dans un mode de réalisation préféré de l'appareil, le dispositif de lavage comprend 4 moyens à emplacement fixe d'application d'un champ magnétique à la partie inférieure des tubes de réaction, lesdits moyens étant constitués chacun par une paire d'aimants 50 permanents, chaque aimant ayant une induction magnétique de 1023 tesla (ou Wb.m⁻) environ. Les aimants ont une dimension correspondant à la surface occupée par deux tubes réactionnels, permettant ainsi d'appliquer un champ magnétique simultanément à deux tubes consécutifs.

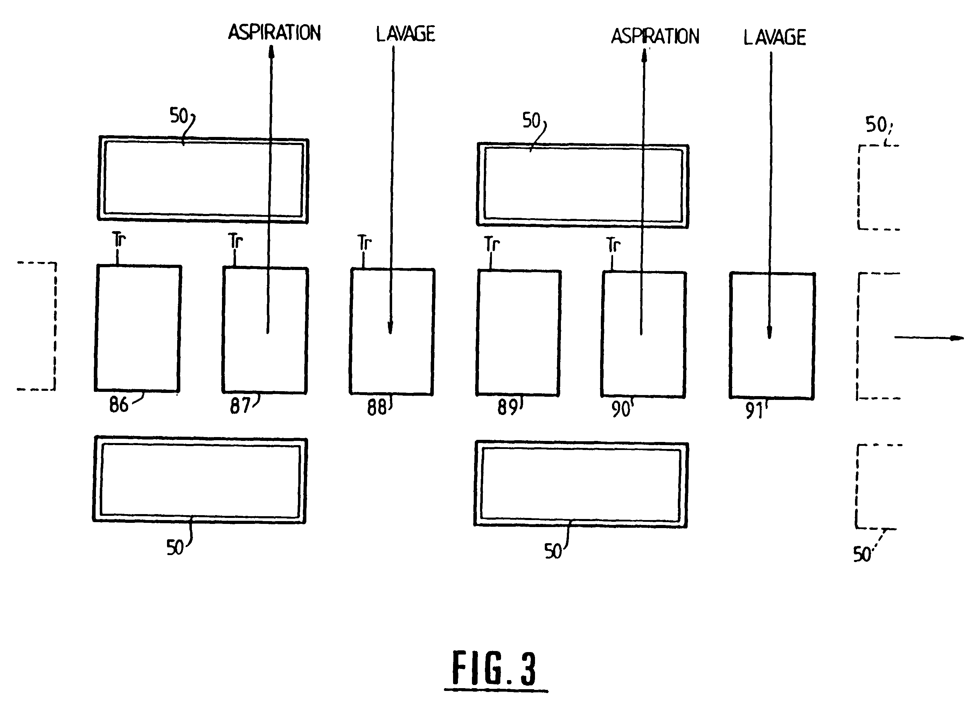

Comme visible sur la figure 3, les deux aimants d'une paire sont disposés face à face et de part et d'autre de deux tubes de réaction. Chaque paire d'aimants est associée à une armature métallique de canalisation des lignes de flux magnétique, permettant de focaliser le champ magnétique sur les deux seuls tubes de réaction amenés par la rotation du module réactionnel entre ladite paire d'aimants, et de soustraire le tube de réaction d'amont et le tube de réaction d'aval au champ magnétique.

Cette figure 3 illustre schématiquement le fonctionnement du dispositif de lavage dans un mode de réalisation préféré de l'appareil selon l'invention, dans lequel ledit dispositif occupe les positions 86 à 98 du module réactionnel, avec les quatres paires d'aimants situées respectivement en positions 86-87, 89-90, 92-93, 95-96 ; les quatre aiguilles d'aspiration situées respectivement en positions 87, 90, 93, 96, les trois aiguilles de distribution de la solution de lavage en position 88, 91, 94, et l'aiguille de distribution de substrat en position 98 du module réactionnel. La durée d'aimantation nécessaire à la sédimentation desdites billes est de l'ordre de 15 secondes.

Lorsqu'un tube est amené en position 86 dudit module, une préaimantation des billes magnétiques est réalisée à hauteur de la première paire d'aimants et permet leur séparation du milieu liquide d'incubation, puis en position 87, à laquelle se trouve la première aiguille d'aspiration, une aimantation des billes magnétiques parachève ladite séparation et une aspiration du liquide contenu dans le tube est réalisée ; en position 88, un lavage des billes magnétiques sédimentées en position 87 est réalisé à l'aide de la première aiguille de lavage ; et la succession préaimantation, aimantation-aspiration, lavage est à nouveau réalisée en positions 89, 90, 91 ; 92, 93, 94, puis une dernière succession comprenant la distribution du substrat à la place du lavage, est réalisée en positions 95, 96, 98.

Un tel dispositif de lavage permet à chaque tube d'être soumis successivement à plusieurs opérations de lavage, dont chacune comprend une préaimantation des billes magnétiques, une aimantation associée à une aspiration du surnageant et une distribution d'une solution de lavage, tout en maintenant une vitesse de rotation constante du module réactionnel.

Le module réactionnel comprend en outre un moyen de lecture approprié de la réaction immunologique, lequel moyen de lecture peut être notamment un photomètre à filtres interférentiels permettant la lecture systématique de chaque tube ou cuvette réactionnelle à trois longueurs d'onde différentes. Le moyen de lecture peut être avantageusement associé à un moyen d'application d'un champ magnétique à la partie inférieure des tubes réactionnels devant être lus.

Un tel appareil permet notamment le dosage de l'hCG, de la TSH, de la T3 totale et libre, de la T4 totale et libre, du cortisol, des IgG ou des IgM présentes en particulier dans les infections suivantes toxoplasmose, rubéole, infections à *Chlamydia,* hépatite, infections à VIH, de polypeptides et protéines tels que AFP, ACE.

Le marqueur peut avantageusement être de la phosphatase alcaline.

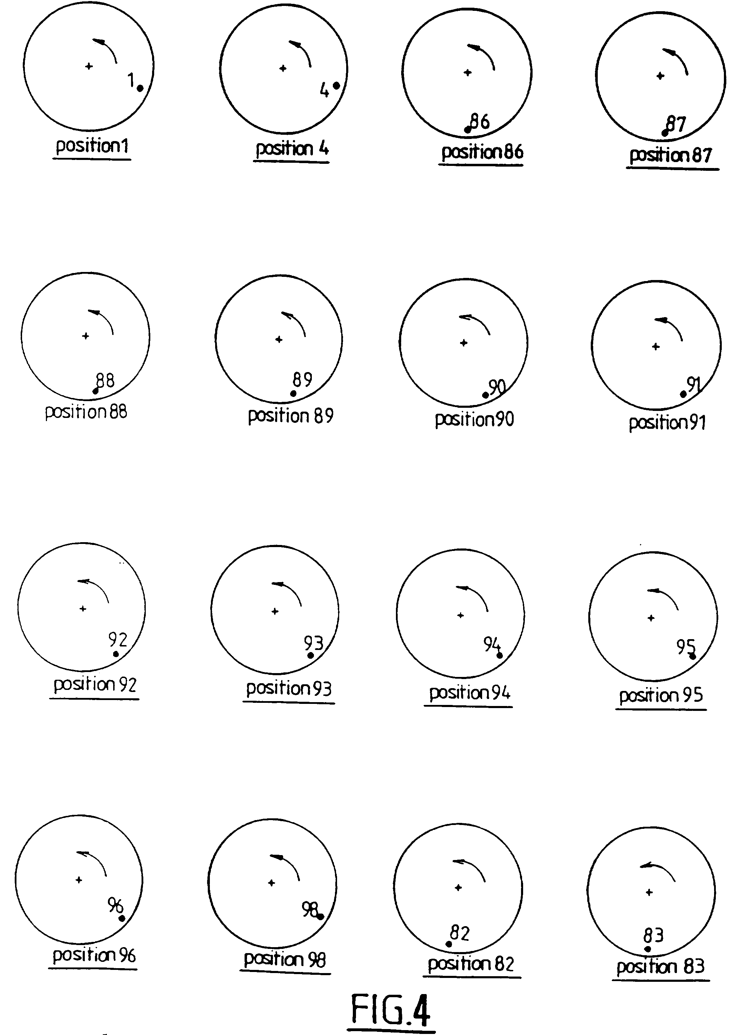

La figure 4 montre la succession des étapes d'un cycle analytique comme défini plus haut et comprenant une incubation immunologique et une incubation enzymatique, dans un mode de réalisation de l'installation comportant 100 tubes au niveau du module réactionnel :
- en position 1, on a le dépôt de l'échantillon à analyser ;
- en position 4, on a le dépôt du réactif ;
- en position 86, on a la première préaimantation ;
- en position 87, on a la première aimantation et aspiration ;
- on a le premier lavage en position 88 ;
- la deuxième série préaimantation, aimantation, aspiration et lavage est réalisée en positions 89,90 et 91 ;
- la troisième série préaimantation, aimantation et aspiration et lavage est réalisée en positions 92, 93 et 94 ;
- la quatrième série comprend la distribution du substrat de la phosphatase alcaline (PNPP) et comprend une préaimantation, une aimantation, une aspiration et une distribution du substrat en positions 95, 96 et 98 ;
- la lecture de la réaction est précédée par une préaimantation en position 82, la lecture proprement dite ayant lieu en position 83 à l'aide d'un système de lecture approprié et notamment d'un photomètre à filtres interférentiels, avec une lecture systématique à trois longueurs d'onde différentes, et ce de manière non limitative.

Cet appareil permet notamment de réaliser des dosages immunoenzymatiques comme suit :

### Exemple 1 : Exemple de mise en oeuvre du procédé de dosage immunologique à l'aide d'un appareil conforme à l'invention : dosages dans un même sérum de l'hCG, de la prolactine, des IgG anti-Chlamydia, des IgG anti-rubéole, des IgG antitoxoplasmose, dénommé ci-après "plateau grossesse".

Dans cet exemple, le dosage des IgG et IgM antitoxoplasmose, des IgG antirubéole, des IgG anti-*Chlamydia* nécessite deux incubations immunologiques, la première en présence d'un premier réactif R₁ constitué de billes magnétiques couplées à une substance S₁ se liant spécifiquement à la substance à détecter, la seconde en présence d'un second réactif R₂ ou "conjugué" et comprenant un marqueur couplé à une substance S₂, identique ou différente de S₁ mais qui ne réagit pas avec S₁, alors que le dosage de l'hCG et de la prolactine ne nécessite qu'une seule incubation immunologique, avec un prémélange des réactifs R₁ et R₂ précités.

Les réactifs R₁, utilisés dans cet exemple, ont été préparés avec des particules de latex de polystyrène, de diamètre compris entre 0,7 et 1,5 µl, renfermant une charge magnétique et dont le rapport masse de matière magnétisable/masse de matière non magnétisable est compris entre 60 et 70 %.

Ces billes, dénommées "Billes ESTAPOR" sont fournies par RHONE-POULENC SPECIALITES CHIMIQUES.

Le couplage de la substance S₁ (anticorps monoclonaux, fragments F(ab')₂ d'anticorps monoclonaux, anticorps polyclonaux, ...) a été réalisé selon le mode opératoire décrit par WOOD et coll. dans Journal of Clinical Chemistry and Clinical Biochemistry, vol. 21, 1983, pp. 789-797.

Les réactifs R₂ ou conjugués ont été préparés selon un mode opératoire analogue à celui décrit par AVRAMEAS dans Immunochemistry, 5, 43, 1969.

Le marqueur choisi est la phosphatase alcaline (PAL). Le substrat de révélation est le para-nitro-phényl phosphate (PNPP).

Le Tableau I suivant présente la composition et la concentration des réactifs utilisés.

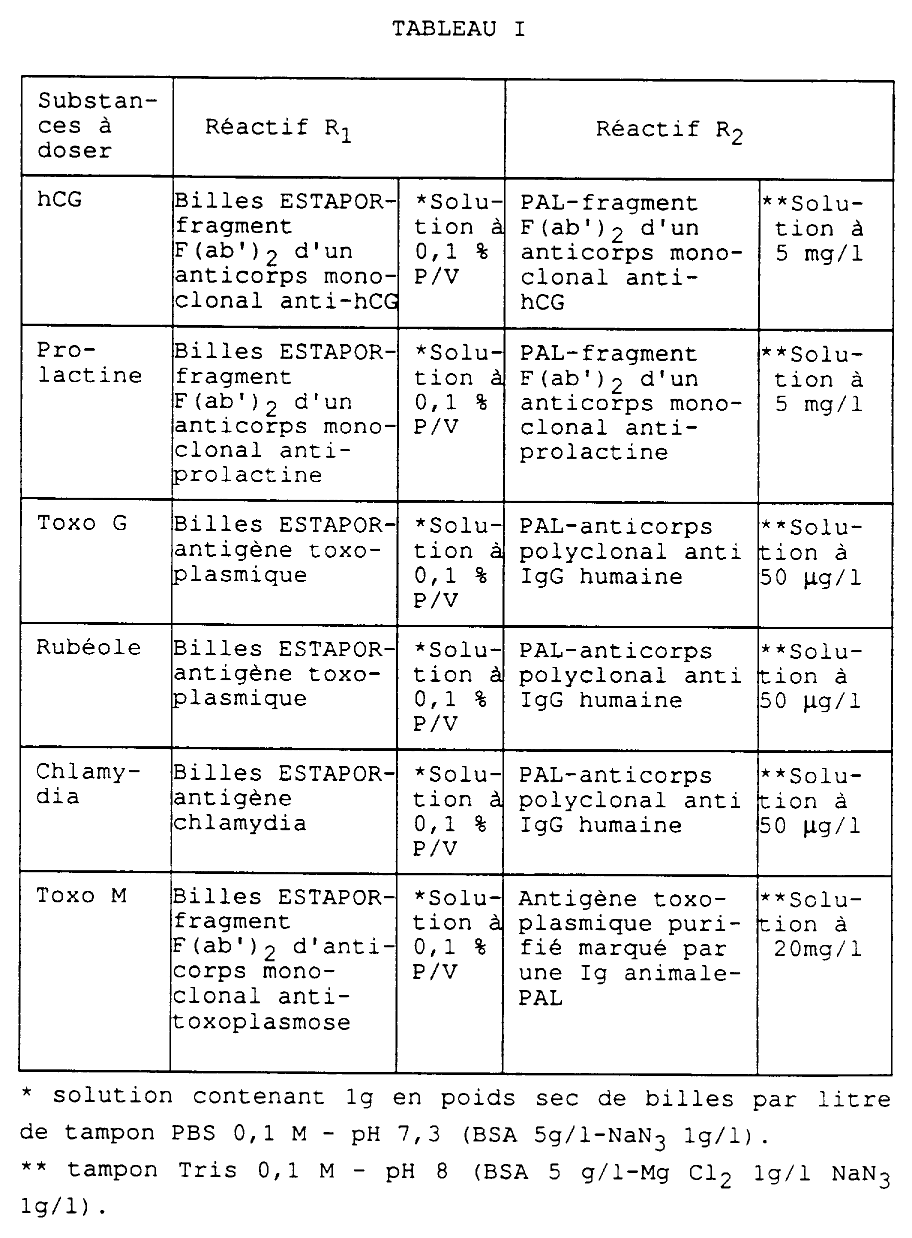

Le tableau II suivant présente les quantités nécessaires exprimées en µl des différents produits à distribuer, en fonction de la substance à doser :

**TABLEAU II**

| Substance | Sérum | Flush | Réactif 1 | Réactif 2 | PNPP |
|---|---|---|---|---|---|
| hCG | 100 | 50 | 300 | | 500 |
| Prolactine | 25 | 50 | 300 | | 500 |
| Toxo G | 10 | 50 | 300 | 400 | 500 |
| Rubéole | 10 | 50 | 300 | 300 | 500 |
| Chlamydia | 10 | 50 | 300 | 400 | 500 |
| Toxo M | 10 | 50 | 300 | 400 | 500 |

Dans cet exemple, le module réactifs E comporte :
- en position 1, le monoréactif hCG comprenant des billes "ESTAPOR" recouvertes d'un fragment F(ab')₂ d'un anticorps monoclonal dirigé contre l'hCG et un conjugué phosphatase alcaline (PAL)-fragment F(ab')₂ d'un anticorps monoclonal dirigé contre l'hCG, différent du premier ;
- en position 2, le monoréactif prolactine comprenant des billes "ESTAPOR" recouvertes d'un fragment F(ab')₂ d'un anticorps monoclonal dirigé contre la prolactine et un conjugué phosphatase alcaline (PAL)-fragment F(ab')₂ d'un anticorps monoclonal dirigé contre la prolactine, différent du premier ;
- en position 3, le réactif R₁ pour le dosage des IgG antitoxoplasmose comprenant des billes "ESTAPOR" recouvertes d'un antigène toxoplasmique ;
- en position 4, le réactif R₄ pour le dosage des IgG antitoxoplasmose, constitué d'un conjugué phosphatase alcaline (PAL)-anticorps polyclonal anti-IgG humaine ;
- en position 5, le réactif R₁ contre la rubéole comprenant des billes magnétiques recouvertes d'un antigène rubéolique ;
- en position 6, le réactif R₂ contre la rubéole, constitué d'un conjugué phosphatase alcaline (PAL)-anticorps polyclonal anti-IgG humaine ;
- en position 7, le réactif R₁anti-*Chlamydia* comprenant des billes magnétiques recouvertes d'un antigène chlamydial ;
- en position 8, le réactif R₂anti-*Chlamydia*, constitué d'un conjugué phosphatase alcaline (PAL)-anticorps polyclonal anti-IgG humaine ;
- en position 9, le réactif R₁pour le dosage des IgM antitoxoplasmose, comprenant des billes magnétiques recouvertes d'un fragment F(ab')₂ d'un anticorps monoclonal anti-toxoplasmose ;
- en position 10, le réactif R₂ pour le dosage des IgM antitoxoplasmose, constitué d'un complexe formé d'un antigène toxoplasmique purifié, marqué par une Ig d'origine animale, conjuguée à la phosphatase alcaline
- et le module échantillons A comprend le tube de sérum à analyser.

Six tubes de réaction vont donc recevoir, tour à tour, en position 1 du module réactionnel C, une quantité appropriée de sérum et une quantité appropriée de "flush" distribuées par le bras B de prélèvement et de distribution des échantillons. Puis, lorsque chacun des tubes arrive en position 4 sur le module réactionnel C, il reçoit une quantité appropriée :
- pour le tube 1, de monoréactif hCG,
- pour le tube 2, de monoréactif prolactine,
- pour le tube 3, de réactif R₁ IgG antitoxoplasmose,
- pour le tube 4, de réactif R₁ IgG antirubéole,
- pour le tube 5, de réactif R₁ IgG antichlamydia,
- pour le tube 6, de réactif R₁ IgM antitoxoplasmose.

Pour les dosages de l'hCG et de la prolactine ne nécessitant qu'une seule incubation immunologique, la séquence analytique se poursuit jusqu'à distribution du substrat lorsque les tubes arrivent en position 98 du module réactionnel, ce qui correspond à l'exécution d'un tour complet de rotation dudit module. Au cours du tour suivant de rotation, se réalisent une préaimantation en position 82 et la lecture de chaque réaction en position 83, à trois longueurs d'onde différentes (405, 450 et 604 nm).

Pour les dosages nécessitant deux incubations immunologiques, la première incubation est réalisée de la même manière que ci-dessus, excepté qu'il n'y a pas de distribution de substrat en position 98 ; en effet, lorsque les tubes arrivent en position 4 du second tour de rotation du module réactionnel C, ils reçoivent respectivement une quantité appropriée :
- pour le tube 3, de réactif R₂ IgG antitoxoplasmose,
- pour le tube 4, de réactif R₂ IgG antirubéole,
- pour le tube 5, de réactif R₂ IgG antichlamydia,
- pour le tube 6, de réactif R₂ IgM antitoxoplasmose,
pour la réalisation d'une seconde incubation immunologique, qui se poursuit jusqu'à distribution du substrat en position 98 du second tour de rotation du module, puis une préaimantation et la lecture du résultat s'effectuent respectivement en position 82 et 83 du troisième tour de rotation dudit module.

Le Tableau III suivant présente le cycle analytique de chacune des substances :

**TABLEAU III**

| POSITIONS DU MODULE REACTIONNEL | | hCG (TUBE 1) | TOXO G (TUBE 3) |
|---|---|---|---|
| | | PROLACTINE (TUBE 2) | TOXO M (TUBE 4) |
| | | | CHLAMYDIA (TUBE 5) |
| | | | RUBEOLE (TUBE 6) |
| 1er tour de rotation du module réactionnel | 1 | Dépôt échantillon + flush | Depôt échantillon + flush |
| | | | |
| | 4 | Dépôt Monoréactif (R₁ + R₂) | Dépôt réactif R₁ |
| | | | |
| | 86 | Préaimantation | Préaimantation |
| | 87 | Aimantation | Aimantation |
| | 88 | Lavage | Lavage |
| | 89 | Préaimantation | Préaimantation |
| | 90 | Aimantation | Aimantation |
| | 91 | Lavage | Lavage |
| | 92 | Préaimantation | Préaimantation |
| | 93 | Aimantation | Aimantation |
| | 94 | Lavage | Lavage |
| | 95 | Préaimantation | Préaimantation |
| | 96 | Aimantation | Aimantation |
| | 98 | Dépôt substrat | |
| | | | |
| 2ème tour de rotation du module réactionnel | 4 | | Dépôt réactif R₂ |
| | | | |
| | 82 | Préaimantation | |
| | 83 | Lecture résultat | |
| | 86 | | Préaimantation |
| | 87 | | Aimantation |
| | 88 | | Lavage |
| | 89 | | Préaimantation |
| | 90 | | Aimantation |
| | 91 | | Lavage |
| | 92 | | Préaimantation |
| | 93 | | Aimantation |
| | 94 | | Lavage |
| | 95 | | Préaimantation |
| | 96 | | Aimantation |
| | 98 | | Dépôt substrat |
| | | | |
| 3ème tour de rotation du module réactionnel | 82 | | Préaimantation |
| | 83 | | Lecture résultat |
| TEMPS DE SORTIE DU 1er RESULTAT | | 30 MINUTES | 47 MINUTES |

Pour un temps d'arrêt des tubes de réaction dans chaque position du module réactionnel C de 10 secondes, le premier résultat est obtenu au bout de 30 minutes pour les dosages ne nécessitant qu'une seule incubation immunologique et au bout de 47 minutes pour les dosages nécessitant deux incubations immunologiques.

Ainsi, le temps nécessaire à la réalisation de 100 dosages nécessitant une incubation immunologique est d'environ 50 minutes et le temps nécessaire à la réalisation de 100 dosages nécessitant deux incubations immunologiques est d'environ 65 minutes.

L'exemple qui vient d'être décrit porte sur le dosage de plusieurs substances différentes présentes dans le même échantillon de sérum. On comprendra qu'il est possible de ne doser, dans un autre échantillon de sérum, qu'une seule substance, telle que la prolactine par exemple. Il s'agit donc bien d'un dosage multiparamétrique à accès aléatoire, permettant de détecter un nombre différent de substances dans chaque échantillon.

Il faut noter que le procédé décrit ci-àessus, mis en oeuvre dans l'appareil conforme à l'invention, a l'avantage de comporter des étapes d'incubation de même durée, quelle que soit la substance recherchée, la concentration de conjugué approprié à chaque substance étant ajustée de manière que les étapes d'incubation soient effectivement de même durée.

### Exemple 2 : dosage de 15 paramètres différents.

Le tableau IV ci-après montre les quantités des différentes substances à ajouter :

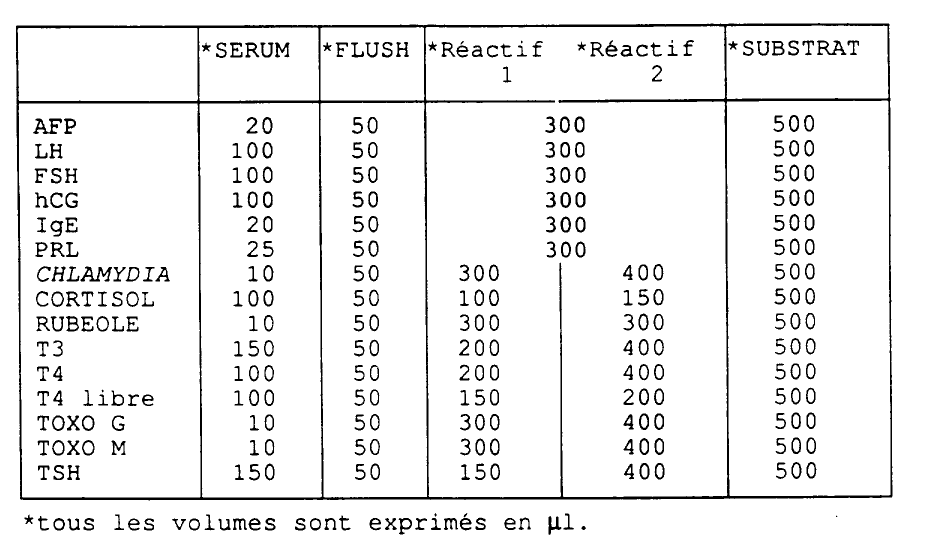

De manière générale, le réactif utilisé pour le dosage des substances qui ne nécessitent qu'une incubation immunologique est constitué par des billes recouvertes d'un fragment F(ab')₂ d'un anticorps monoclonal dirigé contre la substance à doser et d'un conjugué PAL-F(ab')₂ d'un anticorps monoclonal différent du premier, dirigé contre la substance à doser.

Pour ce qui concerne les substances qui nécessitent deux incubations immunologiques :
- cortisol : le premier réactif est constitué de billes recouvertes d'anticorps polyclonaux anti-cortisol et le deuxième réactif est constitué de cortisol marqué à la PAL ;
- T3 totale, T3 libre, T4 totale et T4 libre : le premier réactif est constitué de billes recouvertes d'anticorps monoclonaux anti-T3 ou T4 sous forme F(ab')₂ et le deuxième réactif est constitué de T3 ou T4 marquée à la PAL ;
- TSH : le premier réactif est constitué de billes recouvertes d'anticorps monoclonaux anti-TSH sous forme F(ab')₂ et le deuxième réactif est constitué de fragment F(ab')₂ d'un autre anticorps monoclonal dirigé contre la TSH marquée à la PAL.

Comme dans l'exemple 1, on peut programmer le dosage d'un nombre de paramètres différents pour chaque échantillon à analyser.

### Exemple 3 : Rôle de la masse magnétisable des réactifs, dans la mise en oeuvre du procédé.

Si l'on procède à un dosage tel que décrit à l'exemple 1 ci-dessus avec des billes magnétiques (DYNAL S.A.) ayant les caractéristiques suivantes :

| | |
|---|---|
| Diamètre : | 2,8 µm ± 0,2 µm |
| Coefficient de variation | 5 % (habituellement < 2 %) |
| Masse de matière magnétisable | 12 ± 2 %, |

ces dernières, dont la masse de matière magnétisable est de 12 %, sont aspirées lors du lavage, et en conséquence aucun résultat ne peut être obtenu ; au contraire, les billes magnétiques conformes à l'invention, dont la masse de matière magnétisable est supérieure à 45 %, permettent d'obtenir des résultats dans des délais très courts.

## Revendications

1. Dispositif de lavage utilisable dans la mise en oeuvre d'un procédé de dosage de type immunologique en phase hétérogène, du type comprenant un support solide sous forme de billes magnétiques, caractérisé en ce qu'il comprend :
(1) quatre moyens d'application d'un champ magnétique à la partie inférieure des tubes (Tr) de réaction contenant l'échantillon à analyser et un ou plusieurs réactifs appropriés aux dosages à réaliser, l'un desdits réactifs consistant en billes magnétiques recouvertes d'une substance se liant spécifiquement à la substance à détecter, en suspension dans un liquide approprié et constituées d'une matrice organique renfermant une charge magnétique, lesdites billes ayant un rapport masse de matière magnétisable/masse de matière non magnétisable supérieur ou égal à 45 %, chaque moyen d'application d'un champ magnétique consistant de préférence en une paire d'aimants disposés face à face et de part et d'autre de deux tubes de réactions consécutifs, chaque paire d'aimants (50) étant associée à une armature métallique de canalisation des lignes de flux magnétique, pour que l'application du champ magnétique soit limitée uniquement à deux tubes consécutifs à la fois ; et
(2) une tête de lavage (L) comprenant sur un support :
(i) quatre moyens d'aspiration du liquide contenu dans lesdits tubes,
(ii) trois moyens de distribution d'un liquide de lavage auxdits tubes,
(iii) un moyen de distribution de substrat approprié,
lesdits moyens étant répartis sur ledit support, de telle sorte que le premier moyen soit nécessairement un moyen d'aspiration et le dernier moyen, un moyen de distribution du substrat,
lequel dispositif de lavage étant mobile selon son axe vertical.

2. Appareil de dosage automatique de type immunologique d'au moins une substance dans une pluralité d'échantillons à analyser, en plusieurs étapes successives constituant un cycle analytique, en phase hétérogène, du type comprenant un support solide sous forme de billes magnétiques, lequel appareil comprend :
(1) un module échantillons (A) constitué par une pluralité de supports de tubes (Te) contenant lesdits échantillons ;
(2) un module réactionnel (C) constitué par :
(i) une pluralité de supports de tubes (Tr) destinés à recevoir successivement une quantité aliquote desdits échantillons et une quantité aliquote d'un réactif approprié, et
(ii) une pluralité de positions de traitement séquentielles desdits tubes de réaction, constituant un cycle analytique ;
(3) un module réactifs (E) constitué par une pluralité de supports de tubes contenant le/les réactifs appropriés aux différents dosages à réaliser, au moins l'un desdits réactifs étant sous forme de billes magnétiques recouvertes avec une substance se liant spécifiquement à la substance à détecter, en suspension dans un liquide approprié ;
(4) un moyen de prélèvement et de distribution (B) des échantillons dans les tubes (Tr) dudit module réactionnel ;
(5) un moyen de prélèvement et de distribution (D) des réactifs dans les tubes dudit module réactionnel ;
(6) un moyen approprié de lecture de la réaction effectuée dans le module réactionnel ;
lequel appareil est caractérisé en ce que :
(a) ledit module de réaction (C) connecté à un microprocesseur de contrôle, inclut un dispositif, selon la revendication 1, de lavage desdites billes magnétiques, constituées d'une matrice organique renfermant une charge magnétique, lesdites billes ayant un rapport masse de matière magnétisable/masse de matière non magnétisable supérieur ou égal à 45 %, et
(b) en ce que ledit appareil comprend un système informatique constitué par un ordinateur de commande des différents modules et moyens (1) à (6), et permettant l'exécution d'une succession de cycles analytiques.

3. Appareil selon la revendication 2, caractérisé en ce que les modules réactionnel, échantillons et réactifs comprennent chacun une couronne entraînée en rotation, lesdites rotations des différentes couronnes étant synchronisées par le système informatique.

4. Appareil selon la revendication 2 ou la revendication 3, caractérisé en ce que ledit module réactionnel comprend un nombre de positions de tubes correspondant à la réalisation d'au moins un cycle analytique comportant la série d'étapes successives suivantes :
- dépôt de l'échantillon à analyser en posi tion 1 ;
- dépôt du réactif approprié en position 4 ;
- préaimantation en position 86 ;
- aimantation et aspiration en position 87 ;
- lavage en position 88 ;
- préaimantation en position 89 ;
- aimantation et aspiration en position 90 ;
- lavage en position 91 ;
- préaimantation en position 92 ;
- aimantation et aspiration en position 93 ;
- lavage en position 94 ;
- préaimantation en position 95 ;
- aimantation et aspiration en position 96 ;
- distribution éventuelle du substrat en position 98 ;
- préaimantation en position 82 ;
- lecture du résultat en position 83.

5. Appareil selon l'une quelconque des revendications 2 à 4, caractérisé en ce que le temps d'arrêt d'un tube de réaction dans une position est compris entre 5 et 20 secondes environ.

6. Appareil selon l'une quelconque des revendications 2 à 5, caractérisé en ce que le module réactionnel est, en outre, associé à un dispositif de régulation de la température.

7. Appareil selon l'une quelconque des revendications 2 à 6, caractérisé en ce que l'appareil comprend en outre au moins un moyen de décontamination (101-110) par rinçage des moyens de prélèvement et de distribution (B) et (D) des échantillons et des réactifs.

8. Procédé de dosage immunologique en phase hétérogène, notamment par compétition ou par une méthode bi-site, en plusieurs étapes successives, d'au moins une substance dans une pluralité d'échantillons à analyser, utilisant comme support solide des billes magnétiques appropriées, du type comprenant des étapes :
- de distribution successive d'échantillons et de réactifs,
- d'incubation immunologique,
- de lavage et
- de mesure de ladite substance,
lesquelles étapes étant automatisées, constituant un cycle analytique incluant une pluralité de positions de traitement séquentielles desdits tubes réactifs et étant déterminées par les rotations synchrones de modules échantillons, réactifs et réactionnel, portant respectivement les tubes contenant les échantillons à analyser, les tubes réactifs et les tubes de réaction,
lequel procédé est caractérisé en ce que :
(a) lesdites billes magnétiques, recouvertes d'une substance se liant spécifiquement à la substance à détecter, en suspension dans un liquide approprié, sont constituées d'une matrice organique renfermant une charge magnétique, et ayant un rapport masse de matière magnétisable/masse de matière non magnétisable supérieur ou égal à 45 %,
(b) ladite incubation immunologique est de durée identique quelle que soit la substance à déterminer et est égale à une rotation du module de réaction entre les positions de traitement 5 et 85,
(c) le lavage desdites billes magnétiques est mis en oeuvre à l'aide d'un dispositif de lavage selon la revendication 1, et
(d) l'étape de mesure comprend la séquence suivante de positions de traitement :
position 95 : pré-aimantation
position 96 : aimantation et aspiration
position 98 : distribution du substrat
position 82 : pré-aimantation
position 83 : lecture du résultat.

9. Procédé selon la revendication 8, caractérisé en ce que le cycle analytique comprend une seule incubation immunologique et une incubation de révélation constituée par une incubation enzymatique, lesquelles incubations comprennent les étapes suivantes :
I. incubation immunologique :
a - la distribution d'une quantité aliquote d'échantillon à analyser dans un tube de réaction ;
b - la distribution d'une quantité aliquote de réactif approprié dans ledit tube de réaction ;
c - la mise en contact de l'échantillon et du réactif pendant un temps approprié, correspondant à la durée d'une rotation complète d'un tube de réaction ;
d - une première sédimentation des billes magnétiques suivie d'une aspiration du surnageant puis d'un premier lavage ;
e - une deuxième sédimentation des billes magnétiques suivie d'une aspiration du surnageant puis d'un deuxième lavage ;
f - une troisième sédimentation des billes magnétiques suivie d'une aspiration du surnageant puis d'un troisième lavage ;
g - une quatrième sédimentation des billes magnétiques suivie d'une aspiration du surnageant puis
II. incubation enzymatique
h - une distribution du substrat et la mise en contact des billes magnétiques avec ledit substrat, pendant un temps approprié, dépendant de la durée de rotation dudit tube de réaction entre la distribution du substrat et l'étape de lecture du résultat, suivie d'une sédimentation desdites billes magnétiques ;
i - puis la lecture du résultat ;
chacune desdites étapes correspondant à un nombre de positions séquentielles d'un tube de réaction.

10. Procédé selon la revendication 9, caractérisé en ce que l'incubation est réalisée en présence d'un monoréactif constitué d'un prémélange d'un premier réactif R₁ comprenant des billes magnétiques recouvertes d'une substance S₁ se liant spécifiquement à la substance à détecter et d'un second réactif R₂ appelé "conjugué" constitué par un marqueur approprié couplé à une substance S₂, identique ou différente de S₁ et qui ne réagit pas avec S₁.

11. Procédé selon la revendication 10, caractérisé en ce que ledit monoréactif est constitué d'un prémélange d'un premier réactif R₁ comprenant des billes magnétiques recouvertes d'une substance S₁ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser, et les antigènes appropriés notamment la substance à doser ou l'un de ses fragments et d'un second réactif R₂ ou "conjugué" constitué par de la phosphatase alcaline associée à une substance S₂ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser, et les antigènes appropriés, notamment la substance à doser ou l'un de ses fragments et les complexes antigène-immunoglobuline.

12. Procédé selon la revendication 8, caractérisé en ce que le cycle analytique comprend deux incubations immunologiques et une incubation de révélation constituée par une incubation enzymatique, lequel cycle comprend les étapes suivantes :
I. première incubation immunologique :
a - le dépôt d'une quantité aliquote d'échantillon à analyser dans un tube de réaction ;
b - le dépôt d'une quantité aliquote de réactif approprié dans ledit tube de réaction ;
c - la mise en contact de l'échantillon et du réactif pendant un temps approprié, correspondant à la durée d'une rotation complète d'un tube de réaction ;
d - une première sédimentation des billes magnétiques suivie d'une aspiration du surnageant puis d'un premier lavage ;
e - une deuxième sédimentation des billes magnétiques suivie d'une aspiration du surnageant puis d'un deuxième lavage ;
f - une troisième sédimentation des billes magnétiques suivie d'une aspiration du surnageant puis d'un troisième lavage ;
g - une quatrième sédimentation des billes magnétiques suivie d'une aspiration du surnageant puis
II. deuxième incubation immunologique
- les étapes b- à g- sont réalisées une seconde fois ; puis
III. incubation enzymatique
h - une distribution du substrat et la mise en contact des billes magnétiques avec ledit substrat, pendant un temps approprié, dépendant de la durée de rotation entre la distribution du substrat et l'étape de lecture du résultat, suivie d'une sédimentation des billes magnétiques ;
i - puis la lecture du résultat ; chacune desdites étapes correspondant à un nombre de positions séquentielles d'un tube de réaction.

13. Procédé selon la revendication 12, caractérisé en ce que la première incubation immunologique est effectuée en présence d'un premier réactif R₁ constitué de billes magnétiques recouvertes d'une substance S₁ se liant spécifiquement à la substance à détecter et la seconde incubation immunologique est effectuée en présence d'un second réactif R₂ appelé "conjugué", constitué par un marqueur approprié couplé à une substance S₂, identique ou différente de S₁ et qui ne réagit pas avec S₁.

14. Procédé selon la revendication 13, caractérisé en ce que la première incubation immunologique est effectuée en présence d'un premier réactif R₁, constitué de billes magnétiques recouvertes d'une substance S₁ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser, et les antigènes appropriés notamment la substance à doser ou l'un de ses fragments et la seconde incubation immunologique est effectuée en présence d'un second réactif R₂ ou conjugué constitué par de la phosphatase alcaline associée à une substance S₂ choisie dans le groupe qui comprend les anticorps monoclonaux appropriés, les fragments F(ab')₂ d'un anticorps monoclonal approprié, les anticorps polyclonaux appropriés, lesdits anticorps étant dirigés contre la substance à doser, et les antigènes appropriés notamment la substance à doser ou l'un de ses fragments et les complexes antigène-immunoglobuline.

15. Procédé selon l'une quelconque des revendications 8 à 14, caractérisé en ce qu'il comprend en outre une étape de calibration multiparamétrique, réalisée préalablement au dosage des échantillons.

16. Procédé selon l'une quelconque des revendications 8 à 15, caractérisé en ce que la durée d'une incubation immunologique est sensiblement égale à une rotation complète du module réactionnel, quelle que soit la substance à doser.

## Patentansprüche

1. Spüleinrichtung zur Durchführung eines immunologischen, heterogenphasigen Dosierverfahrens, welche einen festen Träger in Form von magnetischen Kugeln aufweist, **dadurch gekennzeichnet, daß** sie folgendes aufweist:
(1) vier Mittel zum Erzeugen eines magnetischen Feldes in dem unteren Bereich von Reaktionsröhren (Tr), welche die zu analysierenden Proben enthalten, und ein oder mehrere für die Dosierung geeignete Reagenzien, von welchen eines dieser Reagenzien aus magnetischen Kugeln besteht, die von einer Substanz bedeckt sind, die sich spezifisch mit der zu detektierenden Substanz als eine Suspension in einer geeigneten Flüssigkeit verbindet, die eine organische Matrix bilden, welche eine magnetische Ladung umschließt, wobei das Verhältnis von magnetisierbarer zu nicht magnetisierbarer Masse dieser Kugeln größer oder gleich 45% beträgt, jedes Mittel zum Erzeugen eines magnetischen Feldes vorzugsweise aus einem Magnetenpaar besteht, die gegenüberliegend zu beiden Seiten von zwei aufeinanderfolgenden Reaktionsröhren angeordnet sind, und jedes Magnetenpaar (50) eine metallische Umhüllung zur Ausrichtung der magnetischen Feldlinien aufweist, sodaß die Erzeugung des magnetischen Feldes immer nur auf zwei aufeinanderfolgende Röhren begrenzt ist, und
(2) einen Spülkopf(L) welcher an einer Halterung folgendes aufweist:
(i) vier Mittel zum Absaugen der in den Röhren enthaltenen Flüssigkeit,
(ii) drei Mittel zum Verteilen einer Spülflüssigkeit auf diese Röhren,
(iii) ein Mittel zum Verteilen eines geeignetes Substrates,
wobei diese Mittel an der Halterung in der Weise angeordnet sind, daß das erste notwendigerweise ein Mittel zum Absaugen und das letztere notwendigerweise ein Mittel zum Verteilen eines Substrates ist und
die Spülvorrichtung entlang ihrer Vertikalachse bewegbar ist.

2. Vorrichtung zum automatischen immunologischen Dosieren von zumindest einer Substanz in einer Mehrzahl von zu analysierenden heterogenphasigen Proben in mehreren aufeinanderfolgenden Schritten, die einen analytischen Zyklus bilden, welche einen festen Träger in Form magnetischer Kugeln umfaßt und die Vorrichtung weiters folgendes aufweist:
(1) ein Probemodul (A), das aus einer Mehrzahl von Trägern für Röhren (Te) besteht, welche die Proben enthalten,
(2) ein Reaktionsmodul (C), das aus folgendem besteht:
(i) einer Mehrzahl von Trägern für Röhren (Tr), die bestimmt sind, um aufeinanderfolgend eine aliquote Menge der Proben und eine aliquote Menge eines geeigneten Reagens aufzunehmen und
(ii) einer Mehrzahl von sequentiellen Behandlungspositionen dieser Reaktionsröhren, welche einen analytischen Zyklus bilden;
(3) ein Reagenzmodul (E), das aus einer Mehrzahl von Trägern für Röhren besteht, welche das geeignete/die geeigneten Reagenzien für die verschiedenen Dosierungen enthalten, die verwirklicht werden sollen, wobei zumindest eines der Reagenzien in Form von magnetischen Kugeln vorhanden ist, die mit einer Substanz bedeckt sind, welche sich spezifisch mit der zu detektierenden Substanz als Suspension in einer geeigneten Flüssigkeit verbindet;
(4) ein Mittel (B) zum Entnehmen und zum Verteilen der Proben in die Röhren (Tr) des Reaktionsmoduls,
(5) ein Mittel (D) zum Entnehmen und zum Verteilen der Reagenzien in die Röhren des Reaktionsmoduls,
(6) ein geeignetes Mittel zum Ablesen der in dem Reaktionsmodul ablaufenden Reaktion.
und die Vorrichtung, durch folgendes gekennzeichnet ist:
(a) das Reaktionsmodul (C), das mit einem Steuerungsmikroprozessor verbunden ist, weist eine Einrichtung nach Anspruch 1 zum Spülen der magnetischen Kugeln auf, die aus einer organischen Matrix gebildet werden, welche eine magnetische Ladung umgibt, wobei das Verhältnis von magnetisierbarer zu nicht magnetisierbarer Masse dieser Kugeln größer oder gleich 45% ist und
(b) die Vorrichtung weist ein Informationssystem, das aus einem Steuerrechner für die verschiedenen Module besteht, und die Mittel (1) bis (6) auf und ermöglicht das Durchführen einer Aufeinanderfolge analytischer Zyklen.

3. Einrichtung nach Anspruch 2, **dadurch gekennzeichnet, daß** jedes Reaktions-, Proben- und Reagenzienmodul eine in Drehung versetzte Fassung aufweist, wobei die Drehungen der verschiedenen Fassungen durch das Informationssystem synchronisiert sind.

4. Einrichtung nach Anspruch 2 oder 3, dadurch gekennzeichnet, daß das Reaktionsmodul eine Anzahl von Positionen für Röhren aufweist, welche der Verwirklichung zumindest eines analytischen Zyklus entsprechen, welcher die folgende Reihenfolge aufeinanderfolgender Stufen aufweist:
- Aufbewahren der Probe, die analysiert werden soll in Position 1,
- Aufbewahren der geeigneten Reagens in Position 4,
- Vormagnetisieren in Position 86,
- Magnetisieren und Ansaugen in Position 87,
- Spülen in Position 88,
- Vormagnetisieren in Position 89,
- Magnetisieren und Ansaugen in Position 90,
- Spülen in Position 91,
- Vormagnetisieren in Position 92,
- Magnetisieren und Ansaugen in Position 93,
- Spülen in Position 94,
- Vormagnetisieren in Position 95,
- Magnetisieren und Ansaugen in Position 96,
- etwaiges Verteilen des Substrats in Position 98,
- Vormagnetisieren in Position 82,
- Ablesen des Ergebnisses in Position 83.

5. Einrichtung nach irgend einem der Ansprüche 2 bis 4, **dadurch gekennzeichnet, daß** die Haltezeit einer Reaktionsröhre in einer Position ungefähr zwischen 5 und 20 Sekunden liegt.

6. Einrichtung nach irgend einem der Ansprüche 2 bis 5, **dadurch gekennzeichnet, daß** das Reaktionsmodul darüber hinaus einer Einrichtung zum Regeln der Temperatur zugeordnet ist.

7. Einrichtung nach irgend einem der Ansprüche 2 bis 6, **dadurch gekennzeichnet, daß** die Einrichtung darüber hinaus zumindest ein Mittel (101-110) zum Dekontaminieren mittels einer Spülung der Mittel (B) und (D) zum Entnehmen und zum Verteilen der Proben und der Reagenzien aufweist.

8. Verfahren zur immunologischen, heterogenphasigen Dosierung einer Substanz, insbesondere mittels eines Konkurrenzverfahrens oder einer Zweilagen-Methode, in mehreren aufeinanderfolgenden Schritten an einer Mehrzahl von zu analysierenden Proben unter Verwendung von geeigneten magnetischen Kugeln als festen Träger, welches folgende Schritte aufweist:
- aufeinanderfolgende Verteilung von Proben und Reagenzien,
- immunologische Inkubation,
- Spülen und,
- Messen dieser Substanz,
wobei die automatisierten Schritte einen analytischen Zyklus bilden, welcher eine Mehrzahl von sequentiellen Behandlungspositionen der Reaktionsröhren bildet und durch synchrone Rotation von Proben-, Reagenzien- und Reaktionsmodule bestimmt ist, welche die Röhren mit den zu analysierenden Proben tragen, nämlich die Reagenzienröhren und die Reaktionsröhren, und dieses Verfahren durch folgendes gekennzeichnet ist:
(a) die magnetischen Kugeln, welche mit einer Substanz bedeckt sind, die sich spezifisch mit der zu detektierenden Substanz als eine Suspension in einer geeigneten Flüssigkeit verbindet, werden durch eine organische Matrix gebildet, welche eine magnetische Ladung umschließt, und besitzen ein Verhältnis zwischen magnetisierbarer und nicht magnetisierbarer Masse von größer oder gleich 45%,
(b) die Dauer der immunologischen Inkubation ist für alle zu detektierenden Substanzen gleich und entspricht einer Drehung des Reaktionsmoduls zwischen den Behandlungspositionen 5 und 85,
(c) das Spülen dieser magnetischen Kugeln erfolgt mit Hilfe einer Spülvorrichtung nach Anspruch 1 und
(d) der Meßschritt weist die folgende Sequenz von Behandlungspositionen auf:
Position 95: Vor-Magnetisieren,
Position 96: Magnetisieren und Absaugen,
Position 98: Verteilen des Substrates,
Position 82: Vor-Magnetisieren,
Position 83: Ablesen des Ergebnisses.

9. Verfahren nach Anspruch 8, **dadurch gekennzeichnet, daß** der analytische Zyklus eine einzige immunologische Inkubation und eine Entwicklungsinkubation umfaßt, die aus einer enzymatischen Inkubation besteht, wobei diese Inkubationen die folgenden Stufen aufweisen:
I. Immunologische Inkubation:
a) Die Verteilung einer aliquoten Menge einer zu analysierenden Probe in einer Reaktionsröhre,
b) die Verteilung einer aliquoten Menge eines geeigneten Reagens in der Reaktionsröhre,
c) die Herstellung eines Kontakts zwischen Probe und Reagens während einer geeigneten Zeit, welche der Dauer einer vollständigen Drehung einer Reaktionsröhre entspricht,
d) eine erste Sedimentation der magnetischen Kugeln, der eine Ansaugung des Aufschwimmenden, sodann eine erste Spülung folgt,
e) eine zweite Sedimentation der magnetischen Kugeln, der eine Ansaugung des Aufschwimmenden, sodann eine zweite Spülung folgt,
f) eine dritte Sedimentation der magnetischen Kugeln, der eine Ansaugung des Aufschwimmenden, sodann eine dritte Spülung folgt,
g) eine vierte Sedimentation der magnetischen Kugeln, der eine Ansaugung des Aufschwimmenden folgt, sodann
II. Enzymatische Inkubation:
h) Eine Verteilung des Substrats und die Herstellung eines Kontakts der magnetischen Kugeln mit dem Substrat während einer geeigneten Zeit, die von der Dauer einer Drehung der Reaktionsröhre zwischen der Verteilung des Substrats und der Stufe des Ablesens des Ergebnisses abhängt, der eine Sedimentation der magnetischen Kugeln folgt,
i) sodann die Ablesung des Ergebnisses,
wobei jede der Stufen einer Anzahl sequentieller Positionen einer Reaktionsröhre entspricht.

10. Verfahren nach Anspruch 9, **dadurch gekennzeichnet, daß** die Inkubation unter Anwesenheit eines Monoreagens verwirklicht wird, das aus einer Vormischung eines ersten Reagens R1 besteht, das magnetische Kugeln enthält, die von einer Substanz S₁ bedeckt sind, die sich spezifisch mit der Substanz bindet, die erfaßt werden soll, und eines zweiten Reagens R₂, "konjugiert" genannt, das aus einer geeigneten Markierung besteht, die mit einer Substanz S₂ gekoppelt ist, die unterschiedlich oder identisch mit S₁ ist und die mit S₁ nicht reagiert.

11. Verfahren nach Anspruch 10, **dadurch gekennzeichnet, daß** das Monoreagens aus einer Vormischung des folgenden besteht: eines ersten Reagens R₁, das magnetische Kugeln enthält, die von einer Substanz S₁ bedeckt sind, die aus einer Gruppe gewählt ist, die folgendes umfaßt: die geeigneten monoklonalen Antikörper, die Fragmente F (ab')₂ eines geeigneten monoklonalen Antikörpers, die geeigneten polyklonalen Antikörper, wobei die Antikörper gegen die Substanz gerichtet werden, die dosiert werden soll und die geeigneten Antigene, insbesondere die Substanz, die dosiert werden soll oder eines ihrer Fragmente; und eines zweiten Reagens R₂ oder ein "konjugiertes", das aus der alkalischen Phosphatase besteht, die einer Substanz S₂ zugeordnet ist, die aus der Gruppe gewählt ist, welche folgendes umfaßt: die geeigneten monoklonalen Antikörper, die Fragmente F(ab')₂ eines geeigneten monoklonalen Antikörpers, die geeigneten polyklonalen Antikörper, wobei die Antikörper gegen die Substanz gerichtet werden, die dosiert werden soll und die geeigneten Antigene, insbesondere die Substanz, die dosiert werden soll oder eines ihrer Fragmente und die Antigen-Immunoglobulin-Komplexe.

12. Verfahren nach Anspruch 8, **dadurch gekennzeichnet, daß** der analytische Zyklus zwei immunologische Inkubationen und eine Entwicklungsinkubation umfaßt, die aus einer enzymatischen Inkubation besteht, wobei dieser Zyklus die folgenden Stufen aufweist:
I. Erste immunologische Inkubation:
a) Die Aufbewahrung einer aliquoten Menge einer zu analysierenden Probe in einer Reaktionsröhre,
b) die Aufbewahrung einer aliquoten Menge eines geeigneten Reagens in der Reaktionsröhre,
c) die Herstellung eines Kontakts zwischen Probe und des Reagens während einer geeigneten Zeit, welche der Dauer einer vollständigen Drehung einer Reaktionsröhre entspricht,
d) eine erste Sedimentation der magnetischen Kugeln, der eine Ansaugung des Aufschwimmenden, sodann eine erste Spülung folgt,
e) eine zweite Sedimentation der magnetischen Kugeln, der eine Ansaugung des Aufschwimmenden, sodann eine zweite Spülung folgt,
f) eine dritte Sedimentation der magnetischen Kugeln, der eine Ansaugung des Aufschwimmenden, sodann eine dritte Spülung folgt,
g) eine vierte Sedimentation der magnetischen Kugeln, der eine Ansaugung des Aufschwimmenden folgt, sodann
II. Zweite immunologische Inkubation:
die Stufen b) bis g) werden ein zweites Mal ausgeführt, sodann
III. Enzymatische Inkubation:
h) Eine Verteilung des Substrates und die Herstellung eines Kontakts der magnetischen Kugeln mit dem Substrat während einer geeigneten Zeit, die von der Dauer einer Drehung zwischen der Verteilung des Substrats und der Stufe des Ablesens des Ergebnisses abhängt, der eine Sedimentation der magnetischen Kugeln folgt,
i) sodann die Ablesung des Ergebnisses,
wobei jede der Stufen einer Anzahl sequentieller Positionen einer Reaktionsröhre entspricht.

13. Verfahren nach Anspruch 12, **dadurch gekennzeichnet, daß** die erste immunologische Inkubation in Anwesenheit eines ersten Reagens R₁ bewirkt wird, das aus magnetischen Kugeln besteht, die von einer Substanz S₁ bedeckt sind, die sich spezifisch mit der Substanz bindet, die erfaßt werden soll, und die zweite immunologische Inkubation im Beisein eines zweiten Reagens R₂ - "konjugiert" genannt - bewirkt wird, das aus einer geeigneten Markierung besteht, die mit einer Substanz S₂ gekoppelt ist, die unterschiedlich oder identisch mit S₁ ist, und die mit S₁ nicht reagiert.

14. Verfahren nach Anspruch 13, **dadurch gekennzeichnet, daß** die erste immunologische Inkubation in Anwesenheit eines ersten Reagens R1 bewirkt wird, das aus magnetischen Kugeln besteht, die von einer Substanz S1 bedeckt sind, die aus der Gruppe gewählt ist, welche folgendes umfaßt: die geeigneten monoklonalen Antikörper, die Fragmente F (ab')₂ eines geeigneten monoklonalen Antikörpers, die geeigneten polyklonalen Antikörper, wobei die Antikörper gegen die Substanz gerichtet werden, die dosiert werden soll und die geeigneten Antigene, insbesondere die Substanz, die dosiert werden soll oder eines ihrer Fragmente; und die zweite immunologische Inkubation in Anwesenheit eines zweiten Reagens R2 oder eines konjugierten bewirkt wird, das aus der alkalischen Phosphatase besteht, die einer Substanz S2 zugeordnet ist, die aus der Gruppe gewählt ist, welche folgendes umfaßt: die geeigneten monoklonalen Antikörper, die Fragmente F (ab')₂ eines geeigneten monoklonalen Antikörpers, die geeigneten polyklonalen Antikörper, wobei die Antikörper gegen die Substanz gerichtet werden, die dosiert werden soll, und die geeigneten Antigene, insbesondere die Substanz, die dosiert werden soll, oder eines ihrer Fragmente und die Antigen-Immunoglobulin-Komplexe.

15. Verfahren nach irgend einem der Ansprüche 8 bis 14, **dadurch gekennzeichnet, daß** dieses darüber hinaus eine Stufe des Kalibrierens mittels mehrerer Parameter umfaßt, die vor der Dosierung der Proben durchgeführt wird.

16. Verfahren nach irgend einem der Ansprüche 8 bis 15, **dadurch gekennzeichnet, daß** die Dauer einer immunologischen Inkubation unabhängig von der zu dosierenden Substanz im wesentlichen einer vollständigen Drehung des Reaktionsmoduls entspricht.

## Claims

1. Washing device which can be used in the implementation of an assaying method of immunological type in heterogeneous phase, of the type comprising a solid support in the form of magnetic beads, characterized in that it comprises:
(1) four means for applying a magnetic field to the lower part of the reaction tubes (Tr) containing the sample to be analysed, and one or more reagents suitable for the assays to be carried out, one of the said reagents consisting of magnetic beads which are covered with a substance that binds specifically to the substance to be detected, are in suspension in a suitable liquid and consist of an organic matrix containing a magnetic filler, the said beads having a ratio of mass of magnetizable material/mass of nonmagnetizable material greater than or equal to 45%, each means for applying a magnetic field preferably consisting of a pair of magnets arranged facing each other and on either side of two consecutive reaction tubes, each pair of magnets (50) being associated with a metal armature for channelling the magnetic flux lines, so that the application of the magnetic field is limited only to two consecutive tubes at a time; and
(2) a washing head (L) comprising, on a support:
(i) four means for suction of the liquid contained in the said tubes,
(ii) three means for dispensing a washing liquid to the said tubes,
(iii) a means for dispensing a suitable substrate,
the said means being distributed on the said support in such a way that the first means is necessarily a suction means and the last means is necessarily a means for dispensing the substrate,
which washing device is movable along its vertical axis.

2. Apparatus for an automatic assay of the immunological type of at least one substance in a plurality of samples to be analysed, in a plurality of successive steps constituting an analytical cycle, in heterogeneous phase, of the type comprising a solid support in the form of magnetic beads, which apparatus comprises:
(1) a sample module (A) constituted by a plurality of supports for tubes (Te) containing the said samples;
(2) a reaction module (C) constituted by:
(i) a plurality of supports for tubes (Tr) intended successively to receive an aliquot of the said samples and an aliquot of a suitable reagent, and
(ii) a plurality of sequential treatment positions for the said reaction tubes, constituting an analytical cycle;
(3) a reagent module (E) constituted by a plurality of supports for tubes containing the reagent or reagents suitable for the various assays to be carried out, at least one of the said reagents being in the form of magnetic beads which are covered with a substance that binds specifically to the substance to be detected and are in suspension in a suitable liquid;
(4) a means (B) for sampling and dispensing the samples in the tubes (Tr) of the said reaction module;
(5) a means (D) for sampling and dispensing the reagents in the tubes of the said reaction module;
(6) a suitable means for reading the reaction carried out in the reaction module;
which apparatus is characterized in that:
(a) the said reaction module (C), connected to a control microprocessor, includes a device according to Claim 1 for washing the said magnetic beads which consist of an organic matrix containing a magnetic filler, the said beads having a ratio of mass of magnetizable material/mass of nonmagnetizable material greater than or equal to 45%, and
(b) in that the said apparatus comprises a computerized system constituted by a computer for controlling the various modules and means (1) to (6), and allowing a succession of analytical cycles to be executed.

3. Apparatus according to Claim 2, characterized in that the reaction, sample and reagent modules each comprise a ring driven in rotation, the said rotations of the various rings being synchronized by the computerized system.

4. Apparatus according to Claim 2 or Claim 3, characterized in that the said reaction module comprises a number of tube positions corresponding to the carrying out of at least one analytical cycle including the following series of successive steps:
- depositing the sample to be analysed in position 1;
- depositing the suitable reagent in position 4;
- premagnetization in position 86;
- magnetization and suction in position 87;
- washing in position 88;
- premagnetization in position 89;
- magnetization and suction in position 90;
- washing in position 91;
- premagnetization in position 92;
- magnetization and suction in position 93;
- washing in position 94;
- premagnetization in position 95;
- magnetization and suction in position 96;
- optional dispensing of the substrate in position 98;
- premagnetization in position 82;
- reading of the result in position 83.

5. Apparatus according to any one of Claims 2 to 4, characterized in that the time for which a reaction tube stops in one position is between approximately 5 and 20 seconds.

6. Apparatus according to any one of Claims 2 to 5, characterized in that the reaction module is furthermore associated with a device for regulating temperature.

7. Apparatus according to any one of Claims 2 to 6, characterized in that the apparatus furthermore comprises at least one means (101-110) for decontaminating by rinsing the means for sampling and dispensing (B) and (D) the samples and the reagents.

8. Method for immunological assaying in heterogeneous phase, in particular by competition or by a two-site method, in a plurality of successive steps, of at least one substance in a plurality of samples to be analysed, using suitable magnetic beads as a solid support, of the type comprising the steps:
- of successive dispensing of samples and of reagents,
- of immunological incubation,
- of washing, and
- of measuring the said substance,
which steps being automated, constituting an analytical cycle including a plurality of sequential positions for treatment of the said reagent tubes, and being determined by the synchronous rotations of sample, reagent and reaction modules respectively carrying the tubes containing the samples to be analysed, the reagent tubes and the reaction tubes,
which method is characterized in that:
(a) the said magnetic beads, which are covered with a substance that binds specifically to the substance to be detected and are in suspension in a suitable liquid, are constituted by an organic matrix containing a magnetic filler and having a ratio of mass of magnetizable material/mass of nonmagnetizable material greater than or equal to 45%,
(b) the said immunological incubation is of identical duration regardless of the substance to be determined and is equal to one rotation of the reaction module between treatment positions 5 and 85;
(c) washing of the said magnetic beads is implemented with the aid of a washing device according to Claim 1, and
(d) the measurement step comprises the following sequence of treatment positions:
position 95: premagnetization
position 96: magnetization and suction
position 98: dispensing of the substrate
position 82: premagnetization
position 83: reading of the result.

9. Method according to Claim 8, characterized in that the analytical cycle comprises a single immunological incubation and one developing incubation constituted by an enzymatic incubation, which incubations comprise the following steps:
I. immunological incubation:
a - dispensing of an aliquot of sample to be analysed in a reaction tube;
b - dispensing of an aliquot of suitable reagent in the said reaction tube;
c - bringing the sample and the reagent into contact for a suitable period of time, corresponding to the duration of one complete rotation of a reaction tube;
d - a first sedimentation of the magnetic beads followed by suction of the supernatant, then by a first washing;
e - a second sedimentation of the magnetic beads followed by suction of the supernatant, then by a second washing;
f - a third sedimentation of the magnetic beads followed by suction of the supernatant, then by a third washing;
g - a fourth sedimentation of the magnetic beads followed by suction of the supernatant, then
II. enzymatic incubation
h - dispensing of the substrate and bringing the magnetic beads into contact with the said substrate, for a suitable period of time, depending on the duration of rotation of the said reaction tube between dispensing of the substrate and the step of reading the result, followed by sedimentation of the said magnetic beads;
i - then reading of the result;
each of the said steps corresponding to a number of sequential positions of a reaction tube.

10. Method according to Claim 9, characterized in that the incubation is carried out in the presence of a monoreagent constituted by a premix of a first reagent R₁, comprising magnetic beads covered with a substance S₁ that binds specifically to the substance to be detected, and of a second reagent R₂ called the "conjugate" constituted by a suitable label coupled to a substance S₂, identical to or different from S₁, which does not react with S₁.

11. Method according to Claim 10, characterized in that the said monoreagent is constituted by a premix of a first reagent R₁, comprising magnetic beads covered with a substance S₁ chosen from the group comprising suitable monoclonal antibodies, F(ab')₂ fragments of a suitable monoclonal antibody, suitable polyclonal antibodies, the said antibodies being targeted against the substance to be assayed, and suitable antigens, in particular the substance to be assayed or one of its fragments, and of a second reagent R₂ or "conjugate" constituted by alkaline phosphatase associated with a substance S₂ chosen from the group comprising suitable monoclonal antibodies, F(ab')₂ fragments of a suitable monoclonal antibody, suitable polyclonal antibodies, the said antibodies being targeted against the substance to be assayed, and suitable antigens, in particular the substance to be assayed or one of its fragments and antigen-immunoglobulin complexes.

12. Method according to Claim 8, characterized in that the analytical cycle comprises two immunological incubations and one developing incubation constituted by an enzymatic incubation, which cycle comprises the following steps:
I. first immunological incubation:
a - depositing an aliquot of sample to be analysed in a reaction tube;
b - depositing an aliquot of suitable reagent in the said reaction tube;
c - bringing the sample and the reagent into contact for a suitable period of time, corresponding to the duration of one complete rotation of a reaction tube;
d - a first sedimentation of the magnetic beads followed by suction of the supernatant, then by a first washing;
e - a second sedimentation of the magnetic beads followed by suction of the supernatant, then by a second washing;
f - a third sedimentation of the magnetic beads followed by suction of the supernatant, then by a third washing;
g - a fourth sedimentation of the magnetic beads followed by suction of the supernatant, then
II. second immunological incubation
- the steps b- to g- are carried out a second time; then
III. enzymatic incubation
h - dispensing of the substrate and bringing the magnetic beads into contact with the said substrate, for a suitable period of time, depending on the duration of rotation between dispensing of the substrate and the step of reading the result, followed by sedimentation of the magnetic beads;
i - then reading of the result;
each of the said steps corresponding to a number of sequential positions of a reaction tube.

13. Method according to Claim 12, characterized in that the first immunological incubation is carried out in the presence of a first reagent R₁ constituted by magnetic beads covered with a substance S₁ that binds specifically to the substance to be detected, and the second immunological incubation is carried out in the presence of a second reagent R₂ called the "conjugate", constituted by a suitable label coupled to a substance S₂, identical to or different from S₁, which does not react with S₁.

14. Method according to Claim 13, characterized in that the first immunological incubation is carried out in the presence of a first reagent R₁, constituted by magnetic beads covered with a substance S₁ chosen from the group comprising suitable monoclonal antibodies, F(ab')₂ fragments of a suitable monoclonal antibody, suitable polyclonal antibodies, the said antibodies being targeted against the substance to be assayed, and suitable antigens, in particular the substance to be assayed or one of its fragments, and the second immunological incubation is carried out in the presence of a second reagent R₂, or conjugate, constituted by alkaline phosphatase associated with a substance S₂ chosen from the group comprising suitable monoclonal antibodies, F(ab')₂ fragments of a suitable monoclonal antibody, suitable polyclonal antibodies, the said antibodies being targeted against the substance to be assayed, and suitable antigens, in particular the substance to be assayed or one of its fragments and antigen-immunoglobulin complexes.

15. Method according to any one of Claims 8 to 14, characterized in that it furthermore comprises a multiparameter calibration step carried out prior to assaying of the samples.

16. Method according to any one of Claims 8 to 15, characterized in that the duration of an immunological incubation is substantially equal to one complete rotation of the reaction module, irrespective of the substance to be assayed.
